(19) Europäisches Patentamt
European Patent Office
Office européen des brevets

(11) EP 3 246 326 A1

(12) **EUROPEAN PATENT APPLICATION**
published in accordance with Art. 153(4) EPC

(43) Date of publication:
**22.11.2017 Bulletin 2017/47**

(21) Application number: **15877673.2**

(22) Date of filing: **11.12.2015**

(51) Int Cl.:
*C07D 487/04* (2006.01)
*C07D 495/14* (2006.01)
*C07D 487/14* (2006.01)
*C07D 519/00* (2006.01)
*C07F 7/10* (2006.01)
*C07F 9/6561* (2006.01)
*C09K 11/06* (2006.01)
*H01L 51/54* (2006.01)
*H01L 51/46* (2006.01)

(86) International application number:
**PCT/CN2015/097193**

(87) International publication number:
**WO 2016/112762 (21.07.2016 Gazette 2016/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.01.2015 CN 201510015147**

(71) Applicant: **Guangzhou Chinaray Optoelectronic Materials Ltd.**
**Guangzhou, Guangdong 510663 (CN)**

(72) Inventors:
- **PAN, Junyou**
  **Guangzhou**
  **Guangdong 510663 (CN)**
- **HE, Ruifeng**
  **Guangzhou**
  **Guangdong 510663 (CN)**

(74) Representative: **Wibbelmann, Jobst**
**Wuesthoff & Wuesthoff**
**Patentanwälte PartG mbB**
**Schweigerstrasse 2**
**81541 München (DE)**

(54) **COMPOUND, MIXTURE COMPRISING THE SAME, COMPOSITION AND ORGANIC ELECTRONIC DEVICE**

(57) Disclosed in the present invention is a deuterated indolocarbazole compound, mixture comprising said compound, and an organic electronic device. The deuterated indolocarbazole compound optionally replaces a C-H key using a C-D key in an indolocarbazole conjugated structure, and reactivity can be reduced and more preferable chemical and environmental stability is granted to the group. The present invention applies the deuterated indolocarbazole compound to the organic electronic device and provided is a solution for improving a device stability and lifespan.

EP 3 246 326 A1

**Description**

TECHNICAL FIELD

The present disclosure relates to the field of electroluminescent materials and more particularly to a compound (deuterated indolocarbazole compound) and a mixture, a formulation and an organic electronic device comprising the same.

BACKGROUND

Organic light-emitting diodes (OLEDs) have great potential in implementation of novel optoelectronic devices, such as flat panel displays and general lighting applications, because of the synthetic diversity of organic semiconductors, the possibility of achieving large-area flexible devices, low manufacturing cost, and high-performance opto-electronic properties. In order to improve the emitting efficiency of organic light-emitting diodes, a variety of material systems based on fluorescent and phosphorescent materials have been developed. The use of fluorescent materials in organic light-emitting diodes has been highly reliable, but its internal electroluminescence quantum efficiency is limited to 25% under the excitation of electric field, because of the ratio of singlet exciton and triple exciton of 1: 3 according to the quantum statistics. In contrast, organic light-emitting diodes using phosphorescent materials have achieved almost an internal luminescence quantum efficiency of 100%. However, the stability of phosphorescent OLED needs to be improved. Besides the emitter itself, the host material is the key factor for the stability of phosphorescent OLEDs.

Indolocarbazole compounds have the advantages of high carrier transport property, photoelectronic response properties and thermal stability, and therefore become the focus of attention of academia and industry and are widely used in organic light emitting diodes. However, most of the indolocarbazole derivatives currently developed still have poor chemical/environmental stability, mainly because of the lone pairs of electrons of nitrogen atoms in these materials, which are conjugated to the benzene rings to form C-H bonds with high electron cloud density and high reactivity, resulting in poor chemical/environmental stability and short device lifetime of such compounds.

In order to improve the stability of the indolocarbazole compounds and the lifetime of the device, a method for reducing the reactivity of the C-H bond is long sought. One proposal is introduction of an electron-deficient unit that allows the electron cloud to shift toward the electron- deficient unit to reduce the electron cloud density and reactivity of the C-H. However, such method may lead to variations in electrochemical levels at the same time of introduction of the electron-deficient unit. (Adv. Funct. Mater., 2014, 24, 3551-3561).

The evergrowing requirements for material stability and device lifetime have been forcing people to seek for materials with more stable photoelectric properties.

SUMMARY OF THE INVENTION

In various aspects of the present disclosure, there is provided a compound (deuterated indolocarbazole compound), a mixture and a formulation thereof, and the use in organic electronic device. It may solve the problems of stability and short lifetime of the existing indolocarbazole compounds and organic electronic device thereof.

The technical solution provided in one aspect of the present disclosure is as follows: A compound comprising the following general formula (1):

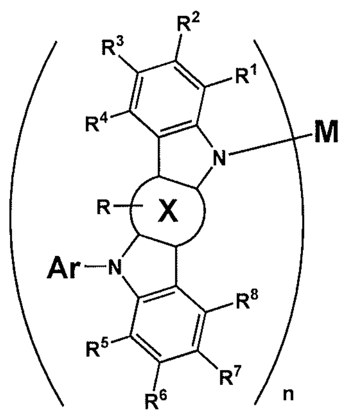

(1)

wherein,

X may be an aromatic ring or a heteroaromatic ring,

R, $R^1$-$R^8$ in each occurence may be the same or different and independently selected from -H, -F, -Cl, Br, I, -D, -CN, -$NO_2$, -$CF_3$, $B(OR^0)_2$, $Si(R^0)_3$, straight chain alkane, alkane ether, alkane sulfide having 1 to 10 carbon atoms, branched alkane, cycloalkane, alkane ether having 3 to 10 carbon atoms; R, $R^1$-$R^8$ may be optionally substituted with one or more active groups $R^0$, and wherein one or more non-adjacent methylene groups may be optionally substituted with $R^0C{=}CR^0$, C=C, $Si(R^0)_2$, $Ge(R^0)_2$, $Sn(R^0)_2$, C=O, C=S, C=Se, $C{=}N(R^0)$, O, S, -COO-, or $CONR^2$; R, $R^1$-$R^8$ H atoms may be optionally substituted with D, F, Cl, Br, I, CN, $NO_2$, one or more active groups $R^0$, aryl, and heteroaromatic ring;

$R^0$ in each occurrence may be the same or different and independently selected from H, D, aliphatic alkanes having 1 to 10 carbon atoms, aromatic hydrocarbon groups, aromatic ring or heteroaromatic ring containing 5 to 10 carbon atoms;

Ar may be selected from alkyl having 1 to 17 carbon atoms, cycloalkyl having 3 to 18 carbon atoms, aromatic hydrocarbon group having 6 to 60 carbon atoms, or aromatic heterocyclic group having 3 to 60 carbon atoms;

M may be selected from aromatic hydrocarbon group having 6 to 60 carbon atoms or aromatic heterocyclic group having 3 to 60 carbon atoms; and

N may be an integer of 1 to 10;

wherein at least one of R, $R^1$-$R^8$ may be a D atom.

In a preferred embodiment, X, in multiple occurrences, may be the same or different group comprising a structure selected from the following:

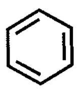 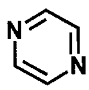 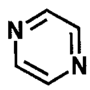 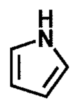 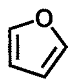 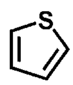

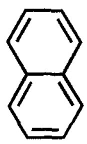 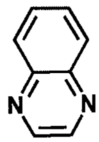 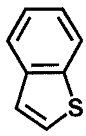 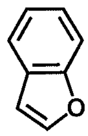 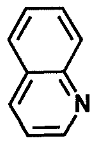

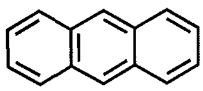 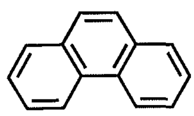 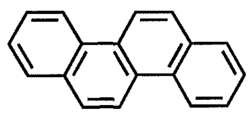

In another preferred embodiment, the compound may have any one structure selected from the following chemical formulas (2) to (7):

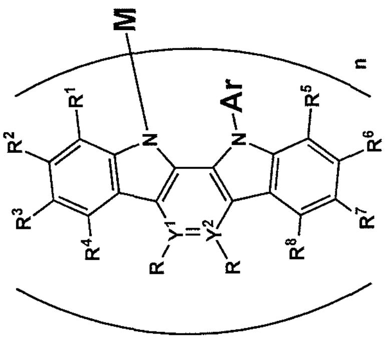

(2)

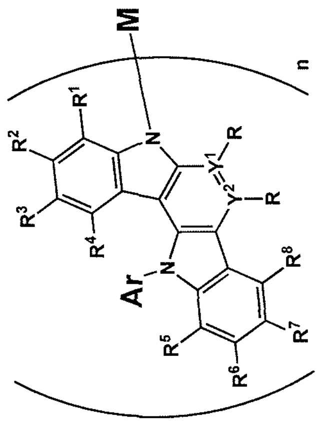

(3)

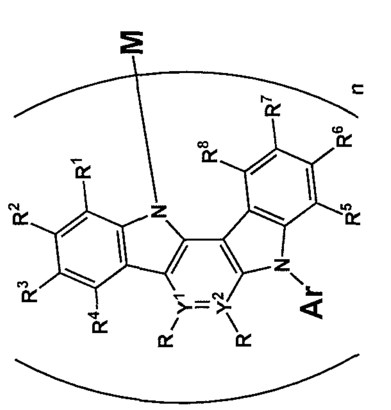

(4)

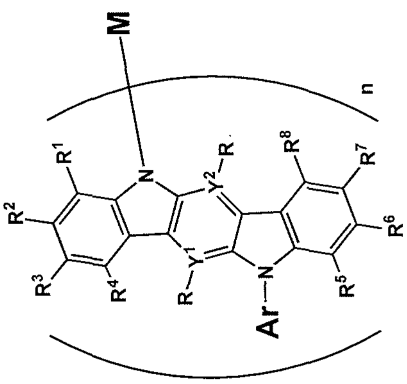

(5)

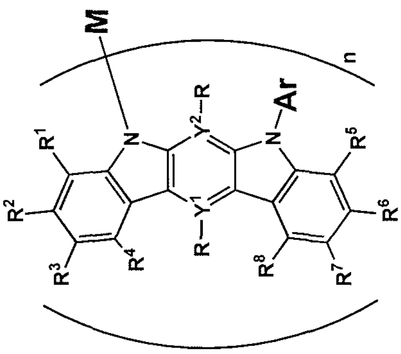

(6)

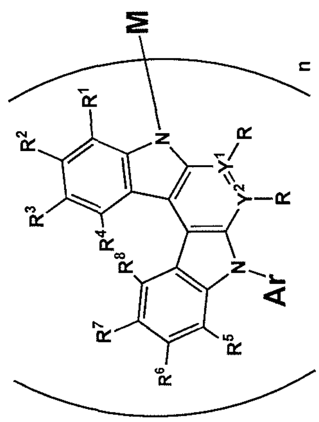

(7)

wherein

$Y^1$ and $Y^2$ may be selected from C or N, respectively, and when $Y^1$ and $Y^2$ may be N, R may be unsubstituted; and Ar may be selected from alkyl having 1 to 17 carbon atoms, cycloalkyl having 3 to 18 carbon atoms, aromatic hydrocarbon group having 6 to 60 carbon atoms, or aromatic heterocyclic group having 3 to 60 carbon atoms; M may be selected from aromatic hydrocarbon group having 6 to 60 carbon atoms or aromatic heterocyclic group having 3 to 60 carbon atoms; and N may be an integer of 1 to 10.

In a particularly preferred embodiment, the compound has a structure of the following chemical formula:

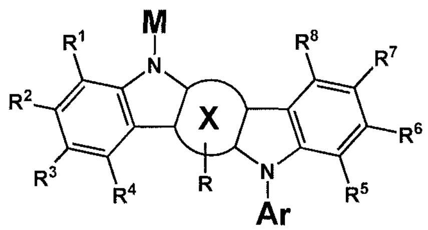

(1a)

Said compound may comprise a group with hole-transport or electron-transport property.

A mixture is provided herein, which may comprise a compound according to any aspect of the present disclosure, and at least one organic functional material which may be selected from the group consisting of: a hole-injection material (HIM), a hole-transport material (HTM), an electron-transport material (ETM), an electron-injection material (EIM), an electron-blocking material (EBM), a hole-blocking material (HBM), a light-emitting material (Emitter), and a host material (Host), or a combination thereof.

A formulation is provided herein, which may comprise a compound according to any aspect of the present disclosure, and at least one organic solvent.

Use of an organic compound according to any aspect of the present disclosure in organic electronic device is also provided herein.

An organic electronic device is provided herein, which may comprise at least one organic compound according to any aspect of the present disclosure, and the mixture thereof

The organic electronic device may be selected from the group consisting of: organic light emitting diode, organic photovoltaic cell, organic light emitting cell, organic field effect transistor, organic light emitting field effect transistor, organic laser, organic spin-electron device, organic sensor, and organic plasmonic emitter diode.

The compounds described in one aspect of the present disclosure are useful in OLEDs, particularly as host materials, to provide longer life. The possible mechanism is as follows, but not limited thereto: the mass of the deuterium (D) atom is twice the mass of the H atom and the C-D bond has low free energy of reaction, reaction rate, and permeation barrier relative to the C-H bond, resulting in low reactivity of the C-D bond relative to the C-H bond (Chem. Rev. 1955, 55, 713-743). This has provided a possibility of improving the chemical/environmental stability of indolocarbazole compounds and optoelectronic devices.

DETAILED DESCRIPTION OF THE EMBODIMENTS

A deuterated indolocarbazole compound according to any aspect of the present disclosure, a mixture and a formulation comprising the same, and their use in an organic electronic device are provided herein. The present disclosure will now be described in greater detail with reference to the accompanying drawings so that the purpose, technical solutions, and technical effects thereof are more clear and comprehensible. It is to be understood that the specific embodiments described herein are merely illustrative of, and are not intended to limit, the disclosure.

In one aspect of the present disclosure, a compound comprising the following general formula (1) is provided:

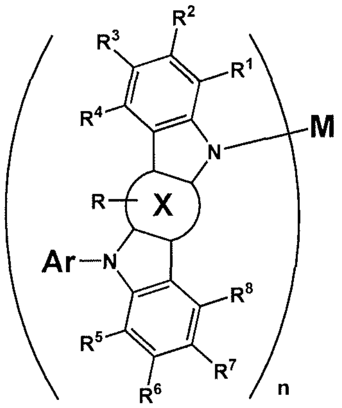

(1)

wherein,

X may be an aromatic ring or a heteroaromatic ring,

R, $R^1$-$R^8$ in each occurence may be the same or different and independently selected from -H, -F, -Cl, Br, I, -D, -CN, $-NO_2$, $-CF_3$, $B(OR^0)_2$, $Si(R^0)_3$, straight chain alkane, alkane ether, alkane sulfide having 1 to 10 carbon atoms, branched alkane, cycloalkane, alkane ether having 3 to 10 carbon atoms; R, $R^1$-$R^8$ may be optionally substituted with one or more active groups $R^0$, and wherein one or more non-adjacent methylene groups may be optionally substituted with $R^0C=CR^0$, C=C, $Si(R^0)_2$, $Ge(R^0)_2$, $Sn(R^0)_2$, C=O, C=S, C=Se, $C=N(R^0)$, O, S, -COO-, or $CONR^2$; R, $R^1$-$R^8$ H atoms may be optionally substituted with D, F, Cl, Br, I, CN, $NO_2$, one or more active groups $R^0$, aryl, and heteroaromatic ring;

$R^0$ in each occurrence may be the same or different and independently selected from H, D, aliphatic alkanes having 1 to 10 carbon atoms, aromatic hydrocarbon groups, aromatic ring or heteroaromatic ring containing 5 to 10 carbon atoms;

Ar may be selected from alkyl having 1 to 17 carbon atoms, cycloalkyl having 3 to 18 carbon atoms, aromatic hydrocarbon group having 6 to 60 carbon atoms, or aromatic heterocyclic group having 3 to 60 carbon atoms;

M may be selected from aromatic hydrocarbon group having 6 to 60 carbon atoms or aromatic heterocyclic group having 3 to 60 carbon atoms; and

N may be an integer of 1 to 10;

wherein at least one of R, $R^1$-$R^8$ is a D atom.

In a preferred embodiment, n is an integer of 1 to 4, more preferably an integer of 1 to 3, even more preferably an integer of 1 to 3, and most preferably an integer of 1 to 2

In a preferred embodiment, the organic compound according to one aspect of the present disclosure is a small-molecular material.

As used herein, the term "small molecule" refers to a molecule that is not a polymer, oligomer, dendrimer, or blend. In particular, there is no repetitive structure in small molecules. The molecular weight of the small molecule is no greater than 3000 g/mole, more preferably no greater than 2000 g/mole, and most preferably no greater than 1500 g/mole.

As used herein, the term "polymer" includes homopolymer, copolymer, and block copolymer. In addition, in the present disclosure, the polymer also includes dendrimer. The synthesis and application of dendrimers are described in Dendrimers and Dendrons, Wiley-VCH Verlag GmbH & Co. KGaA, 2002, Ed. George R. Newkome , Charles N. Moorefield, Fritz Vogtle.

The term "conjugated polymer" as defined herein is a polymer whose backbone is predominantly composed of the sp2 hybrid orbital of carbon (C) atom. Some known non-limiting examples are: polyacetylene and poly (phenylene vinylene), on the backbone of which the C atom can also be optionally substituted by other non-C atoms, and which is still considered to be a conjugated polymer when the sp2 hybridization on the backbone is interrupted by some natural

defects. In addition, the conjugated polymer in the present disclosure may also comprise aryl amine, aryl phosphine and other heteroarmotics, organometallic complexes, and the like.

A first feature of the compound according to one aspect of the disclosure is that: at least one H atom of the organic compound is optionally substituted with deuterium. Preferably, at least one of R, $R^1$-$R^8$ is a D atom.

The present disclosure shows that the deuterated compound has a more preferred stability and that the OLED containing the deuterated compound has a longer lifetime. A possible mechanism is as follows, but is not limited thereto. A reaction involving a C-H bond usually has a reaction rate 6 to 10 times faster than the corresponding C-D bond due to the isotopic effect of the kinetics. Therefore, when the OLED is working, the OLED containing the deuterated compound has a relatively slow decay in the of the decay dynamics.

In some embodiments, those H with higher reactivity may be optionally substituted with deuterium.

In some embodiments, the organic compound may have at least one H atom of Ar optionally substituted with deuterium.

In some embodiments, the organic compound may have at least one H atom of M optionally substituted with deuterium.

Deuterium has a natural abundance of 0.0156% in the ocean, that is, one of 6420 hydrogen atoms is deuterium. The deuterium content in the compounds described in one aspect of the present disclosure may be much greater than the natural abundance. In general at least 1% of the H atoms may be optionally substituted with deuterium, and more preferably, at least 10% of the H atom is optionally substituted with deuterium.

In a preferred embodiment, deuterated organic compound is provided, wherein 20% or above, more preferably 30% or above, even more preferably 40% or above, and most preferably 50% or above of H atoms may be optionally substituted with deuteriume.

In a preferred embodiment, the compound of formula (1) is provided, wherein X, in multiple occurances, may be the same or different group comprising a structure selected from the following:

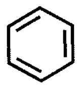
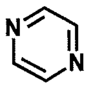
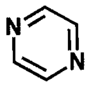
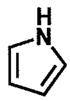
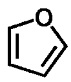
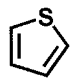

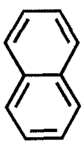
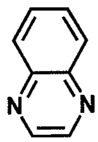
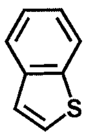
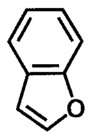
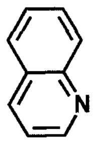

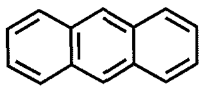
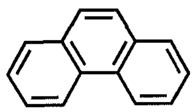
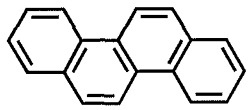

In a preferred embodiment, the compound according to one aspect of the present disclosure may comprise a structure with any one of the following formulas (2) to (7):

| | |
|---|---|
| 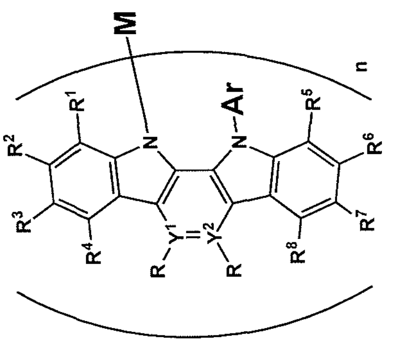 | (2) |
| 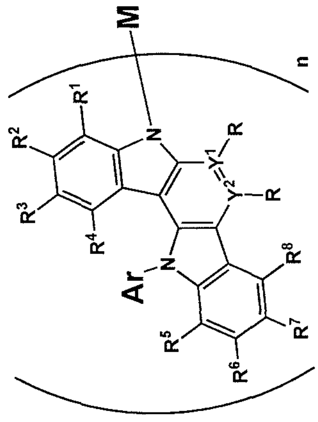 | (3) |
| 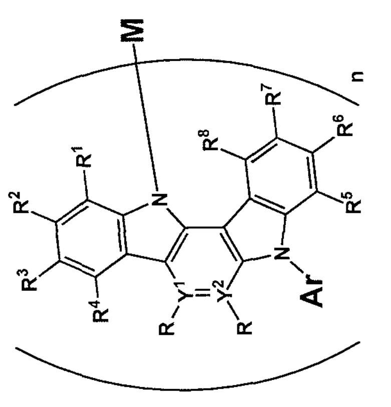 | (4) |
| 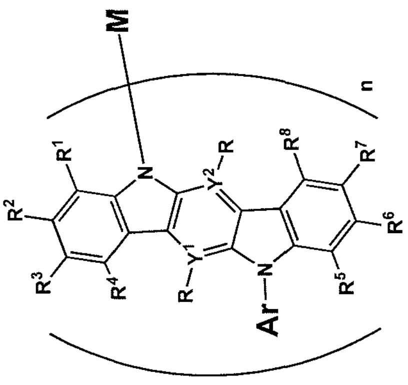 | (5) |
| 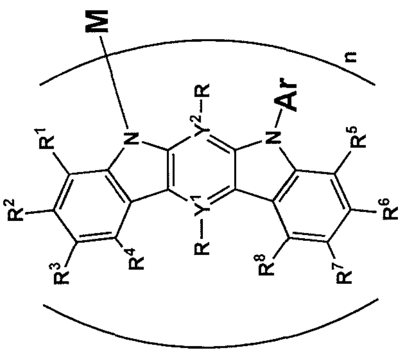 | (6) |
| 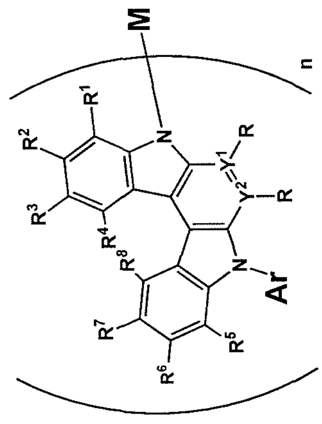 | (7) |

wherein

$Y^1$ and $Y^2$ are selected from C or N, respectively; when $Y^1$ and $Y^2$ are N, there is no substituent on $Y^1$ and $Y^2$; and $Y^1$ and $Y^2$ are preferably C; and

R, $R^1$-$R^8$ may be independently H, D, alkyl, arylalkyl, alkenyl, alkynyl, nitrile, amine, nitro, acyl, alkoxy, carbonyl, sulfone, cycloalkyl or hydroxyl; wherein at least one of R, $R^1$-$R^8$ is a D atom. The alkyl may be an alkyl group having 1 to 17 carbon atoms, a cycloalkyl group having 3 to 18 carbon atoms, and the preferred alkyl group may be a methyl group or a tert-butyl group; and the aromatic alkyl group may be an aryl group having 6 to 60 carbon atoms or a aromatic heterocyclic group having 3 to 60 carbon atoms, including the following non-limiting examples: benzene, naphthalene, anthracene, phenanthrene, pyrene, pyridine, pyrimidine, triazine, fluorene, thiofluorene, silicon fluorene, carbazole, thiophene, furan, thiazole, triphenylamine, triphenylphosphine oxide, tetraphenyl silicon, spirofluorene, spirofluorene, and the like; and the preferred aromatic alkyl groups are phenyl, triazine, carbazole, and pyridine. The number of the deuterium atoms is an integer of 1 to 10, and the preferred optional substitution position is the ortho and para position of the nitrogen atom in indolocarbazole, and the preferred number of optional substitution is an integer of 1 to 6.

Ar may be an alkyl group having 1 to 17 carbon atoms, a cycloalkyl group having 3 to 18 carbon atoms, an aryl group having 6 to 60 carbon atoms, or a aromatic heterocyclic group having 3 to 60 carbon atoms. Preferable examples may include the following non-limiting examples: benzene, naphthalene, anthracene, phenanthrene, pyrene, pyridine, pyrimidine, triazine, fluorene, thiofluorene, silicon fluorene, carbazole, thiophene, furan, thiazole, triphenylamine, triphenylphosphine oxide, tetraphenyl silicon, spirofluorene, spirofluorene, and the like. More preferred groups may be benzene, pyridine, pyrimidine, triazine, carbazole, and the like. One or more of the carbon atoms of the alkyl and cycloalkyl groups may be optionally substituted with heteroatoms such as nitrogen, oxygen, sulfur, silicon and the like. The aryl group and the aromatic heterocyclic group may be formed by a plurality of aromatic groups liked together and may have a substituent, wherein the plurality of aromatic groups are preferably biphenyl, terphenyl, phenylpyridine, diphenyl triazine, phenylcarbazole, pyridyl carbazole and the like. Preferred substituents are fluorine, methyl, t-butyl, methoxy, acetyl and the like.

M may be an aryl group having 6 to 60 carbon atoms or a aromatic heterocyclic group having 3 to 60 carbon atoms. Preferred examples thereof include the following nonlimiting examples: benzene, naphthalene, anthracene, phenanthrene, pyrene, pyridine, pyrimidine, triazine, fluorene, thiofluorene, silicon fluorene, carbazole, thiophene, furan, thiazole, triphenylamine, triphenylphosphine oxide, tetraphenyl silicon, spirofluorene, spirofluorene, and the like. The aryl group or the aromatic heterocyclic group may have a substituent, wherein the preferred substituents may be fluorine, alkyl having 1 to 4 carbon atoms, and alkoxy having 1 to 2 carbon atoms.

N may be an integer of 1 to 10, and n is preferably an integer of 1 to 4.

In a preferred embodiment, the compound according to one aspect of the present disclosure may have a structure represented by the following chemical formula

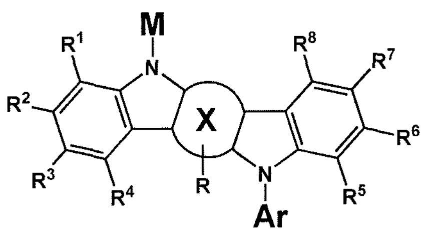

(1a)

In a particularly preferred embodiment, the compound according to one aspect of the present disclosure may comprise a structure represented by any one of the following chemical formulas (2a) to (7a)

| | | |
|---|---|---|
| 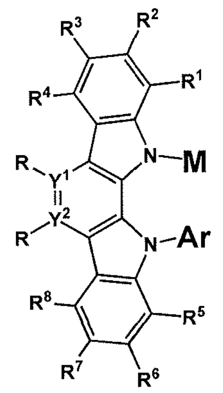 | 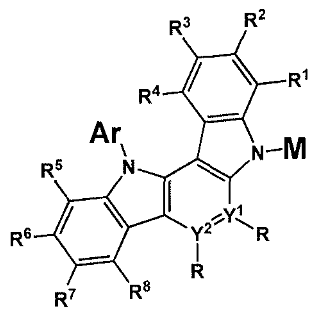 | 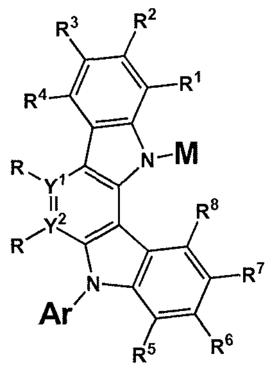 |
| (2a) | (3a) | (4a) |
| 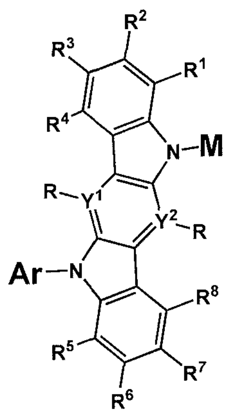 | 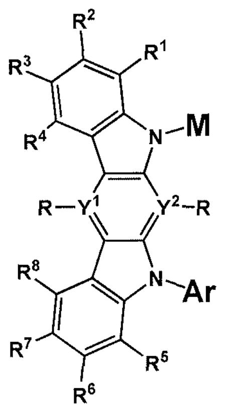 | 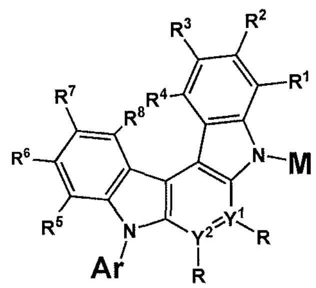 |
| (5a) | (6a) | (7a) |

wherein the symbols are defined as defined in (2) - (7).

In a preferred embodiment, the compound according to one aspect of the present disclosure is provided, wherein at least one M or Ar may comprise a group having a hole-transport property. Suitable groups with hole-transport properties will be described in the hole-transport material (HTM) below.

In another preferred embodiment, the compound according to one aspect of the present disclosure is provided, wherein at least one M or Ar may comprise a group having electron-transport properties. Suitable groups with electron-transport properties will be described in the electron-transport material (ETM) below.

In a particularly preferred embodiment, the compound according to one aspect of the present disclosure is provided, wherein at least one of M or Ar may comprise a group having electron-transport properties and the other may comprise a group having hole-transport properties.

Specific examples of the deuterated indolocarbazole compounds described in accordance with one aspect of the present disclosure are given below, but the deuterated indolocarbazole compounds of the present disclosure are not limited thereto.

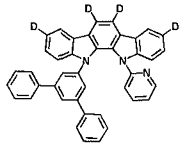 (2-1) , 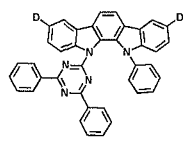 (2-2) , 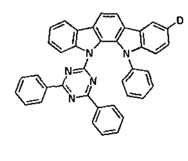 (2-3) , 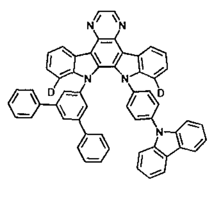 (2-4) ,

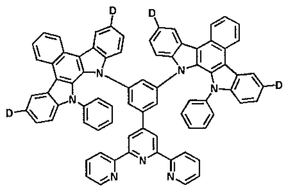 , (2-5) 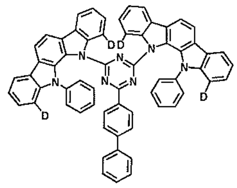 (2-6) , 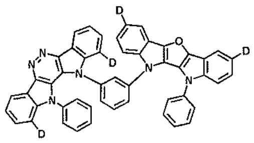 (2-7) ,

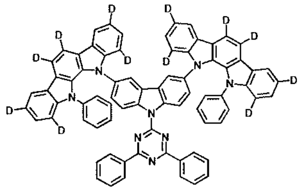 (2-8) , 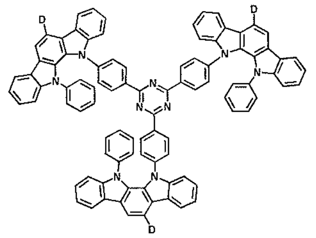 (2-9) ,

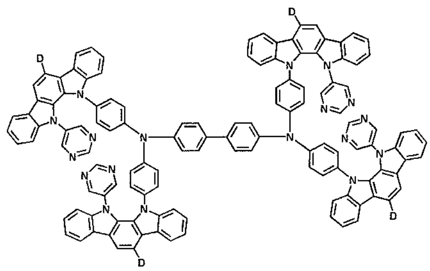 (2-10) , 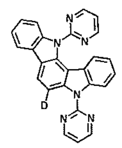 (3-1) , 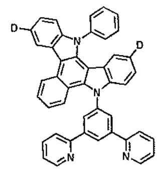 (3-2) ,

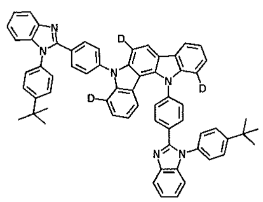 (3-3), 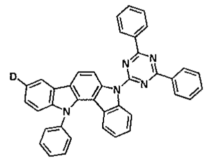 (3-4), 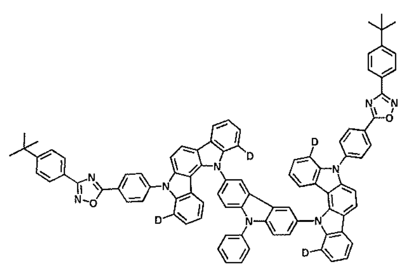 (3-5)

, 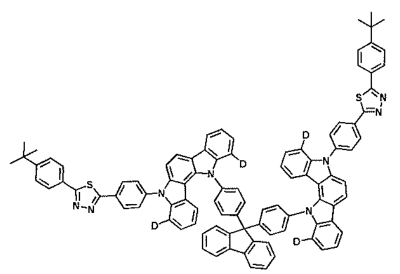 (3-6) , 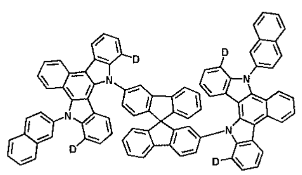 (3-7) , 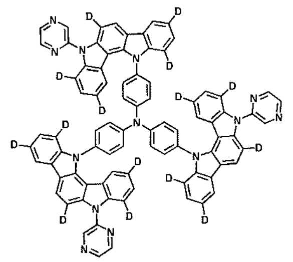

(3-8) , 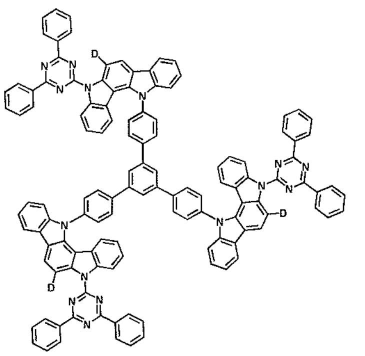 (3-9) , 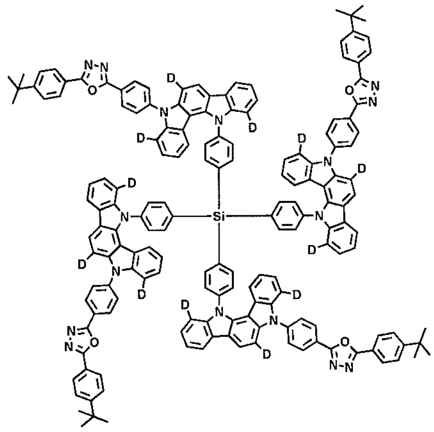 (3-10) ,

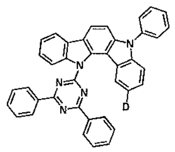 (4-1), 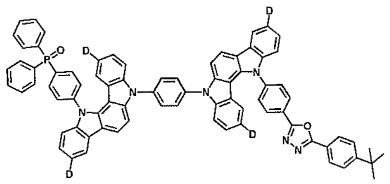 (4-2), 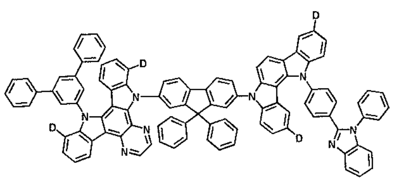 (4-3)

, 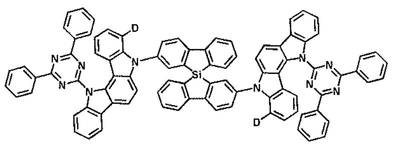 (4-4), 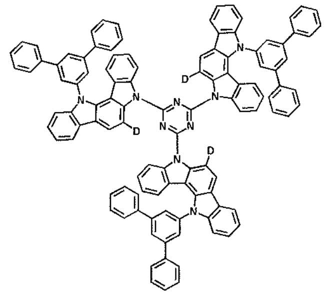 (4-5), 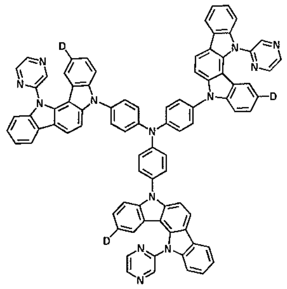

(4-6) , 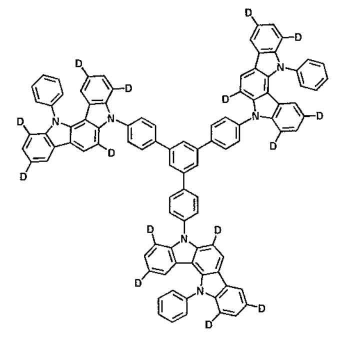 (4-7) , 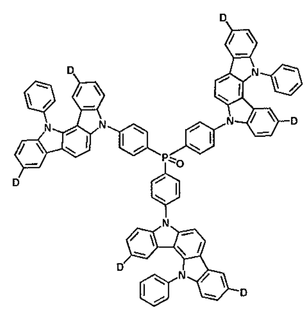 (4-8) ,

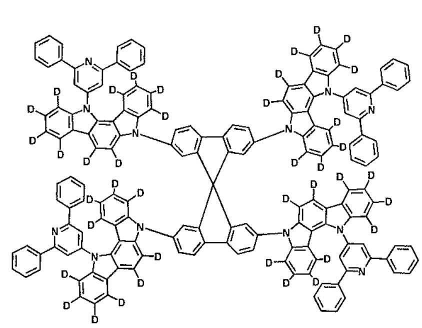 (4-9), 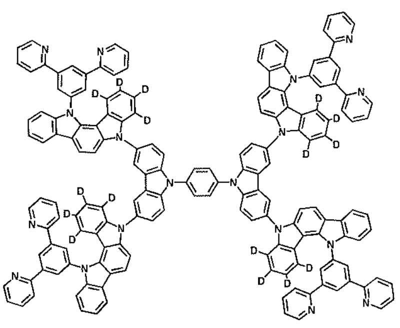 (4-10), 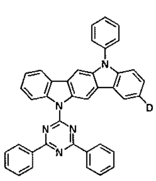 (5-1)

, 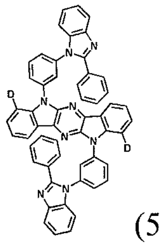 (5-2), 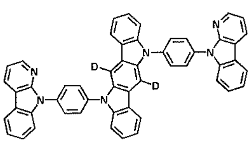 (5-3), 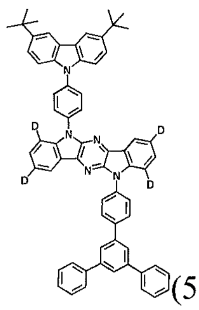 (5-4), 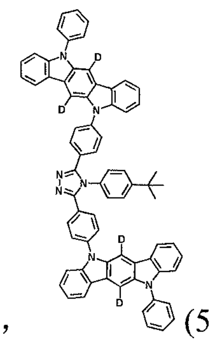 (5-5), 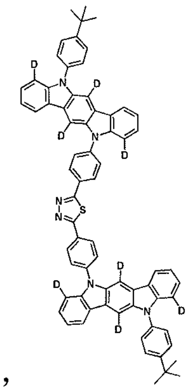

(5-6), 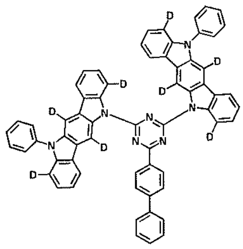 (5-7), 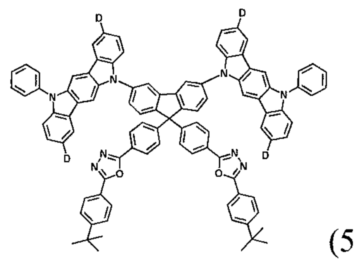 (5-8), 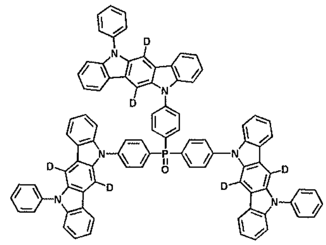 (5-9)

, 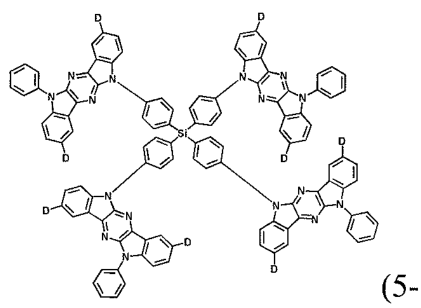 (5-10), 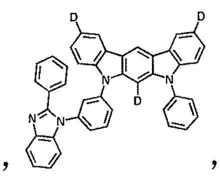 , (6-1) 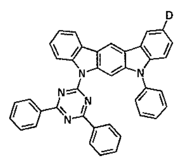 (6-2), 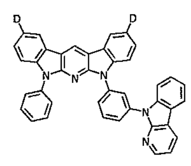

(6-3) , 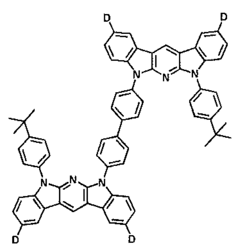 (6-4) , 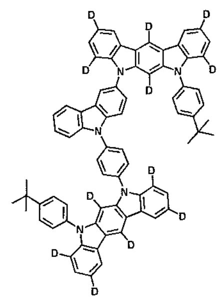 (6-5) , 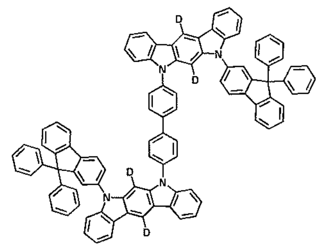 (6-6) ,

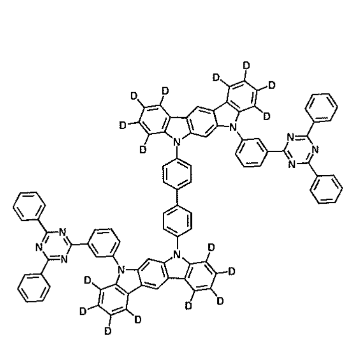 (6-7), 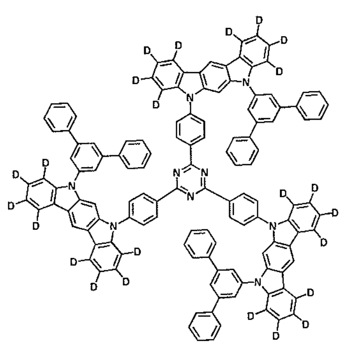 (6-8), 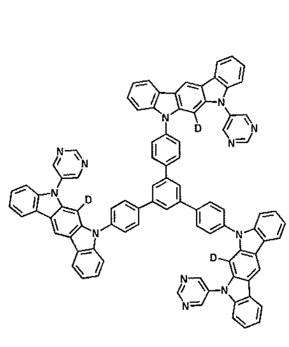 (6-9),

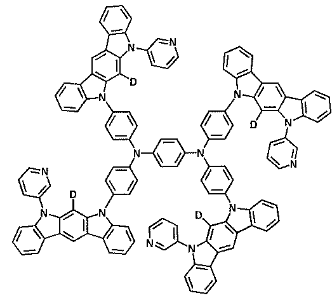 (6-10), 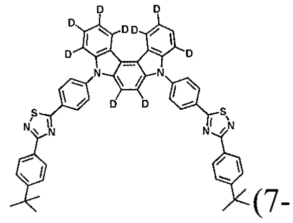 (7-1), 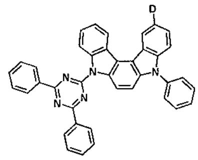 (7-2), 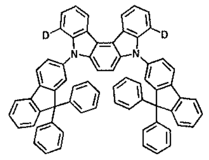

(7-3), 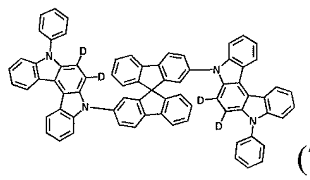 (7-4), 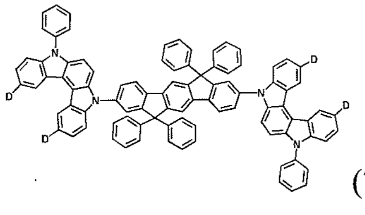 (7-5), 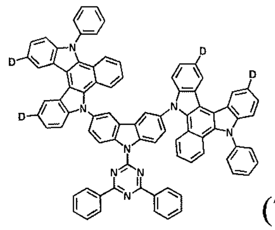 (7-6)

, 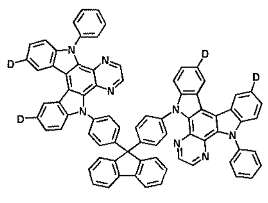 (7-7) , 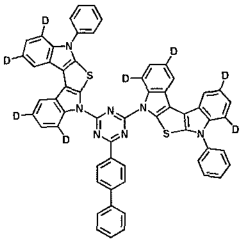 (7-8) , 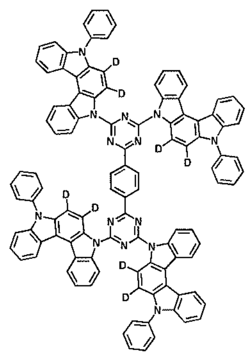 (7-9) ,

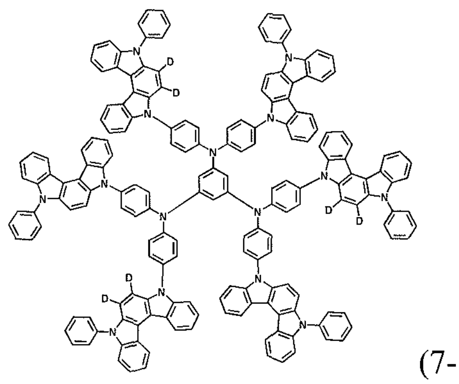 (7-10)

According to one aspect of the present disclosure, There are generally two methods for introducing D atoms into the indolocarbazole conjugated skeleton for synthesizing the deuterated indolocarbazole compounds.

One method may be: preparing an indolocarbazole precursor with a halogen atom, then performing optional substitutaion of functional groups on the N-H bond, and then replacing the the halogen atoms with D atoms. The other method may be: first performing optional substitutaion of functional groups on the N-H bond, then performing optional substitutaion of halogen atoms on the unit, and then replacing the the halogen atoms with D atoms to form the final deuterated indolocarbazole compounds.

The present disclosure also provides a mixture which may comprise an organic compound according to one aspect of the disclosure, and at least another organic functional material.

The organic functional material may include any one or any combination of hole (also referred to as electron hole) injection or transport material (HIM/HTM), hole-blocking material (HBM), electron-injection or transport material

(EIM/ETM), electron-blocking material (EBM), organic host material (Host), singlet emitter (fluorescent emitter), multiplet emitter (phosphorescent emitter), especially light-emitting organometallic complexes. Non-limiting examples of various organic functional materials are described, for example, in WO2010135519A1, US20090134784A1, and WO 2011110277A1..

The organic functional material may be a small-molecule polymeric material.

In the present disclosure, the host material, the matrix material, Host, and Matrix have the same meaning and are interchangeable in use.

In the present disclosure, the metal organic complex, the metal organic complex and the organometallic complex have the same meaning and are interchangeable.

The following is a more detailed description the organic functional material (but not limited thereto).

1. HIM/HTM/EBM

Suitable organic HIM/HTM materials for use in one aspect of the present disclosure may include any one or any combination of the compounds having the following structural units: phthalocyanines, porphyrins, amines, aryl amines, biphenyl triaryl amines, thiophenes, thiophenes such as dithiophenethiophene and thiophthene, pyrrole, aniline, carbazole, indeno-fluorene, and derivatives thereof. Other suitable HIMs also include: fluorocarbon-containing polymers; polymers comprising conductive dopants; conductive polymers such as PEDOT/PSS; self-assembled monomers such as compounds comprising phosphonic acid and sliane derivatives; metal oxides, such as MoOx; metal complex, and a crosslinking compound, or a combination thereof.

The electron-blocking layer (EBL) used in one aspect of the present disclosure is typically used to block electrons from adjacent functional layers, particularly light emitting layers. In contrast to a light-emitting device without a blocking layer, the presence of EBL usually results in an increase in luminous efficiency. The electron-blocking material (EBM) of the electron-blocking layer (EBL) requires a higher LUMO than the adjacent functional layer, such as the light emitting layer. In a preferred embodiment, the EBM has a greater level of excited energy than the adjacent luminescent layer, such as a singlet or triplet level, depending on the emitter. In another preferred embodiment, the EBM has a hole-transport function. HIM/HTM materials, which typically have high LUMO levels, can be used as EBM.

Examples of cyclic aromatic amine-derived compounds that can be used as HIM or HTM include, but are not limited to, the general structure as follows:

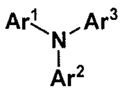 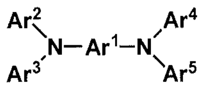 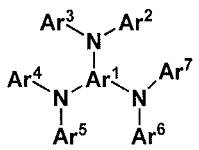 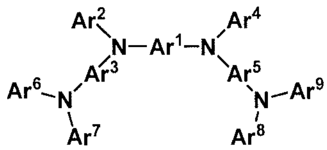 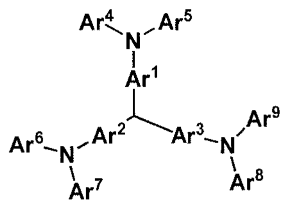

wherein each $Ar^1$ to $Ar^9$ may be independently selected from: cyclic aryl groups such as benzene, biphenyl, triphenyl, benzo, naphthalene, anthracene, phenalene, phenanthrene, fluorene, pyrene, chrysene, perylene, azulene; and aromatic heterocyclic groups such as dibenzothiophene, dibenzofuran, furan, thiophene, benzofuran, benzothiophene, benzoselenophene, carbazole, pyrazole, imidazole, triazole, isoxazole, thiazole, oxadiazole, oxatriazole, dioxazole, thiadiazole, pyridine, pyridazine, pyrimidine, pyrazine, triazine, oxazine, oxathiazin, oxadiazine, indole, benzimidazole, indoxazine, bisbenzoxazole, benzisoxazole, benzothiazole, quinoline, isoquinoline, cinnoline, quinazoline, quinoxaline, naphthalene, phthalein, pteridine, xanthene, acridine, phenazine, phenothiazine, phenoxazine, dibenzoselenophene, benzoselenophene, benzofuropyridine, indolocarbazole, pyridylindole, pyrrolodipyridine, furodipyridine, benzothienopyridine, thienodipyridine, benzoselenophenopyridine, and selenophenodipyridine; groups comprising 2 to 10 ring structures which may be the same or different types of cyclic aryl or aromatic heterocyclic group and are bonded to each other directly or through at least one of the following groups, for example: oxygen atom, nitrogen atom, sulfur atom, silicon atom, phosphorus atom, boron atom, chain structure unit, and aliphatic cyclic group; and wherein each Ar may be further optionally substituted, and the substituents may optionally be hydrogen, alkyl, alkoxy, amino, alkene, alkyne, aralkyl, heteroalkyl, aryl and heteroaryl.

In one aspect, $Ar^1$to $Ar^9$may be independently selected from the group consisting of:

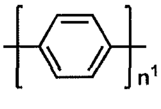 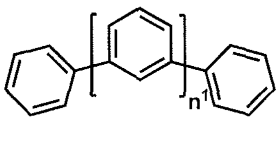 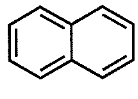 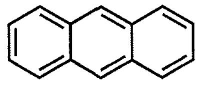 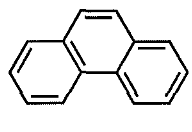

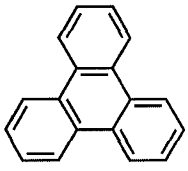 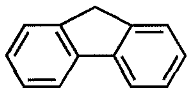 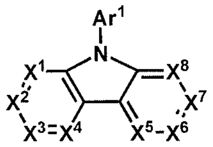 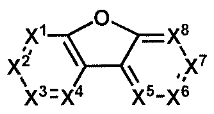 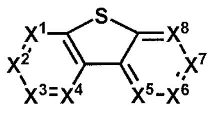

wherein n is an integer of 1 to 20; $X^1$ to $X^8$are CH or N; $Ar^1$is as defined above.

Additional non-limiting examples of cyclic aryl amine-derived compounds may be found in US3567450, US4720432, US5061569, US3615404, and US5061569.

Non-limiting examples of metal complexes that may be used as HTM or HIM include, but are not limited to, the general structure as follows:

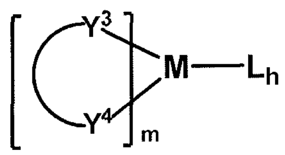

M may be metal having an atomic weight greater than 40;

$(Y^3-Y^4)$ is a bidentate ligand, wherein $Y^3$ and $Y^4$ are independently selected from C, N, O, P, and S; L is an auxiliary ligand; m is an integer from 1 to the maximum coordination number of the metal; m + n is the maximum coordination number of the metal.

In one embodiment, $(Y^3-Y^4)$ may be a 2-phenylpyridine derivative.

In another embodiment, $(Y^3-Y^4)$ may be a carbene ligand.

In another embodiment, M may be selected from Ir, Pt, Os, and Zn.

In another aspect, the HOMO of the metal complex is greater than -5.5 eV (relative to the vacuum level).

Suitable non-limiting examples of HIM/HTM/EBM compounds are set forth in the following table:

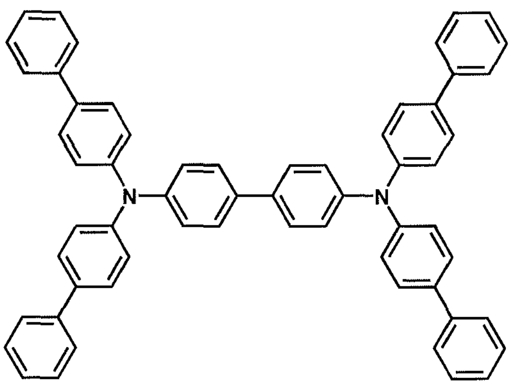 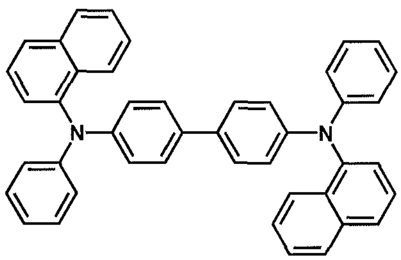

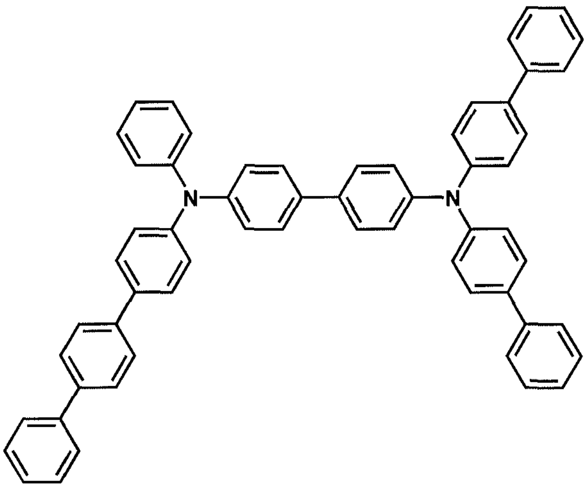
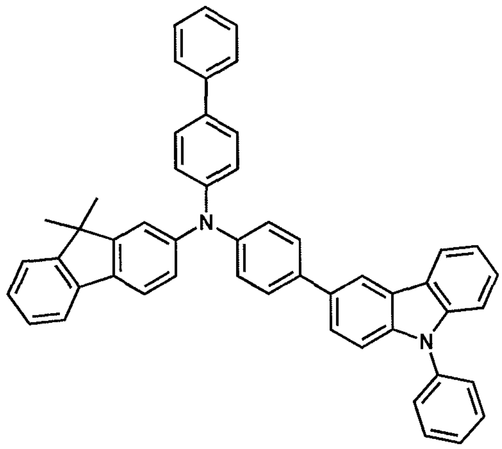

2. EIM/ETM/HBM

Examples of EIM/ETM material used in one aspect of the present disclosure are not particularly limited, and any metal complex or organic compound may be used as EIM/ETM as long as they can transfer electrons. Preferred organic EIM/ETM materials may be selected from the group consisting of tris (8-quinolinolato) aluminum (A1Q3), phenazine, phenanthroline, anthracene, phenanthrene, fluorene, bifluorene, spiro-bifluorene, phenylene-vinylene, triazine, triazole, imidazole, pyrene, perylene, trans-indenofluorene, cis-indenonfluorene, dibenzol-indenofluorene, indenonaphthalene, benzanthracene and their derivatives.

The hole-blocking layer (HBL) used in one aspect of the present disclosure is typically used to block holes from adjacent functional layers, particularly light-emitting layers. In contrast to a light-emitting device without a barrier layer, the presence of HBL usually leads to an increase in luminous efficiency. The hole-blocking material (HBM) of the hole-blocking layer (HBL) requires a lower HOMO than the adjacent functional layer, such as the light-emitting layer. In a preferred embodiment, the HBM has a greater energy level of excited state than the adjacent light-emitting layer, such as a singlet or triplet, depending on the emitter. In another preferred embodiment, the HBM has an electron-transport function. Typically, EIM/ETM materials with deep HOMO levels may be used as HBM.

In another aspect, compounds that may be used as EIM/ETM/HBM may be molecules comprising at least one of the following groups:

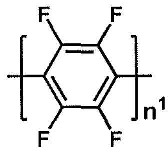
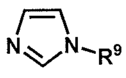
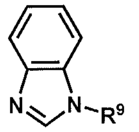
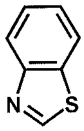
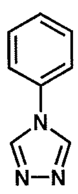
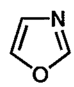
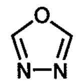
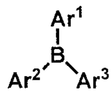

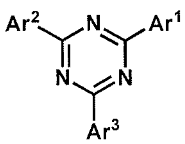
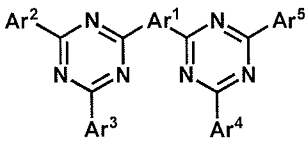
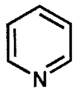
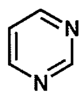
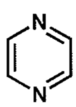
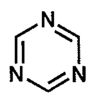
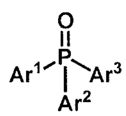
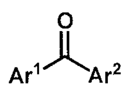

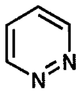
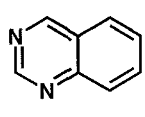
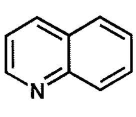
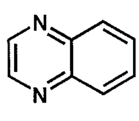
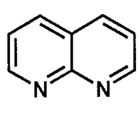
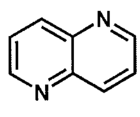
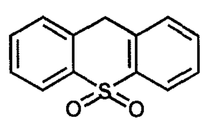

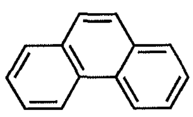
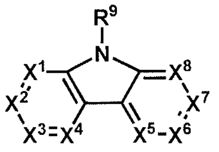
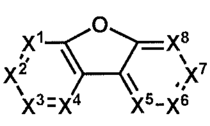
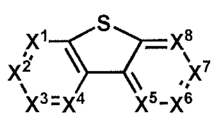
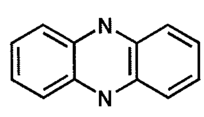

wherein $R^9$ may be selected from the group consisting of: hydrogen, alkyl, alkoxy, amino, alkene, alkyne, aralkyl, heteroalkyl, aryl and heteroaryl, wherein, when they are aryl or heteroaryl, they may have the same meaning as $Ar^1$ and $Ar^2$ in HTM as described above; $Ar^1$ - $Ar^5$ and $X^1$ - $X^8$ may have the same meaning as $Ar^1$ - Arts and $X^1$ - $X^8$ in HTM as described above.

$n^1$ may be an integer from 1 to 20.

On the other hand, examples of metal complexes that may be used as EIM/ETM may include, but are not limited to, the following general structure:

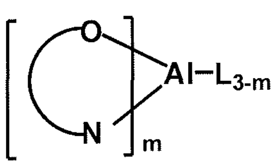
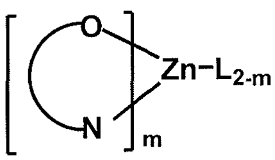
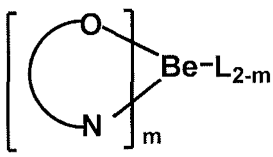
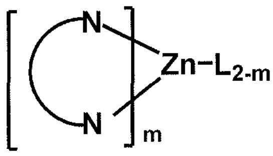

(O-N) or (N-N) is a bidentate ligand, wherein the metal coordinates with O, N, or N, N; L is an auxiliary ligand; and m is an integer whose value is from 1 to the maximum coordination number of the metal.

Non-limiting examples of suitable ETM compounds are listed in the following table:

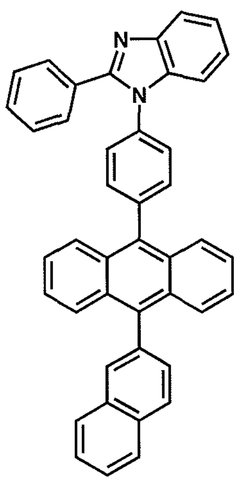
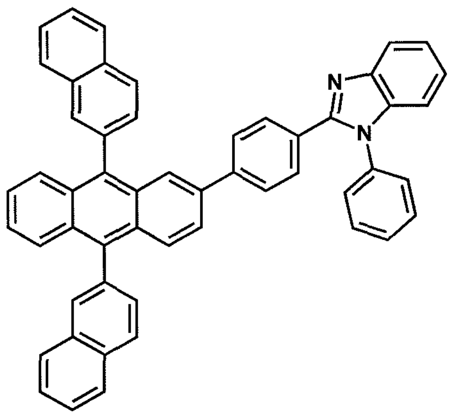
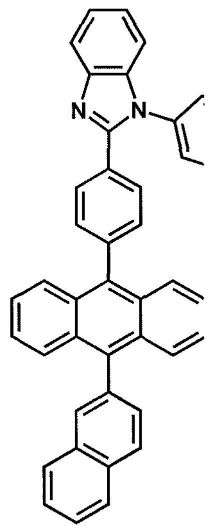

In another preferred embodiment, the organic alkali metal compound may be used as the EIM. In the present disclosure, the organic alkali metal compound may be understood as a compound having at least one alkali metal, i.e., lithium, sodium, potassium, rubidium, and cesium, and further comprising at least one organic ligand.

Non-limiting examples of suitable organic alkali metal compounds may include the compounds described in US 7767317 B2, EP 1941562B1 and EP 1144543B1.

The preferred organic alkali metal compound may be a compound of the following formula:

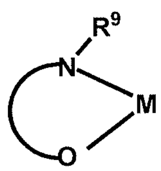

wherein $R^9$ has the same meaning as described above, and the arc represents two or three atoms and the bond to form a 5- or 6-membered ring with metal M when necessary, while the atoms may be optionally substituted with one or more $R^9$; and wherein M is an alkali metal selected from lithium, sodium, potassium, rubidium, and cesium.

The organic alkali metal compound may be in the form of a monomer, as described above, or in the form of an aggregate, for example, two alkali metal ions with two ligands, 4 alkali metal ions and 4 ligands, 6 alkali metal ions and 6 ligand, or in other forms.

The preferred organic alkali metal compound may be a compound of the following formula:

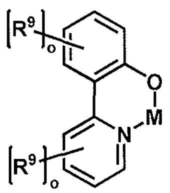 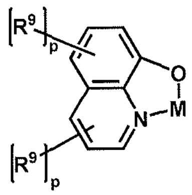 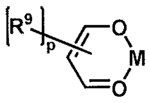

wherein, the symbols used are as defined above, and in addition:

o, each time it may be the same or different, selected from 0, 1, 2, 3 or 4; and

p, each time it may be the same or different, selected from 0, 1, 2 or 3.

In a preferred embodiment, the alkali metal M is selected from the group consisting of lithium, sodium, potassium, more preferably lithium or sodium, and most preferably lithium.

In a preferred embodiment, the organic alkali metal compound is comprised in the electron-injection layer, and more preferably the electron-injection layer consists of the organic alkali metal compound.

In another preferred embodiment, the organic alkali metal compound is doped into other ETMs to form an electron-transport layer or an electron-injection layer, more preferably an electron-transport layer.

Non-limiting examples of a suitable organic alkali metal compound for EIM are listed in the following table:

| | | | | |
|---|---|---|---|---|
| 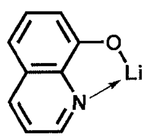 | 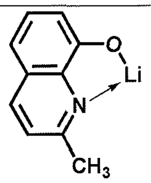 | 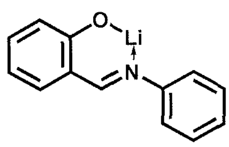 | 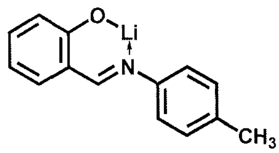 | 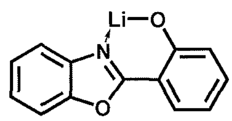 |
| 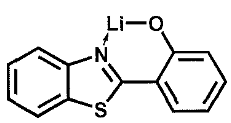 | 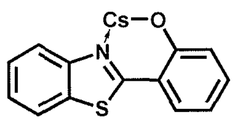 | 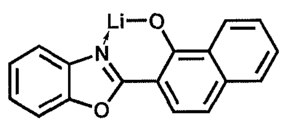 | 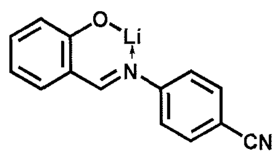 | |

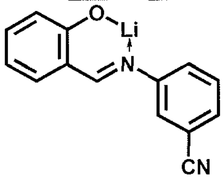
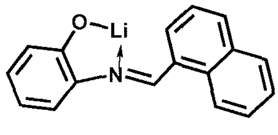
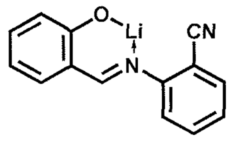
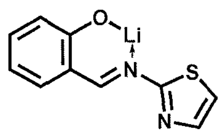
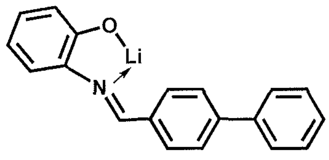
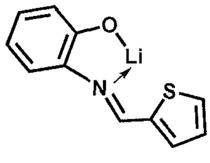
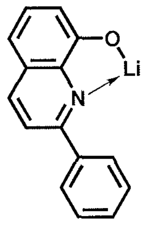
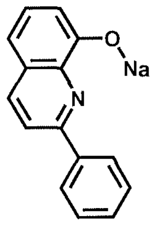
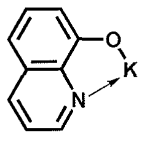
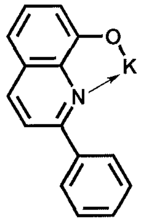
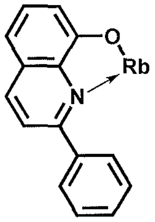
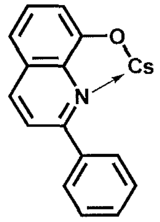
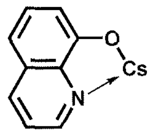
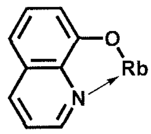
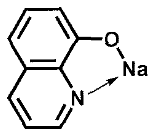

3. Triplet Host Materials:

Examples of a triplet host material used in one aspect of the present disclosure are not particularly limited and any metal complex or organic compound may be used as the host material as long as its triplet energy is greater than that of the light emitter, especially a triplet emitter or phosphorescent emitter.

Examples of metal complexes that may be used as triplet hosts may include, but are not limited to, the general structure as follows:

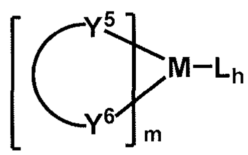

wherein M may be a metal; ($Y^5$-$Y^6$) may be a bidentate ligand, $Y^5$ and $Y^6$ may be independently selected from C, N, O, P, and S; L may be an auxiliary ligand; m may be an integer with the value from 1 to the maximum coordination number of the metal; and, m + n is the maximum number of coordination of the metal.

In a preferred embodiment, the metal complex which may be used as the triplet host has the following form:

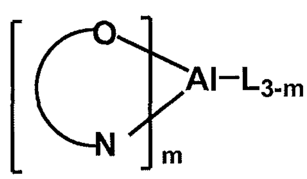
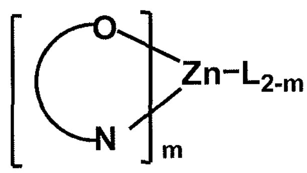

(O-N) may be a bidentate ligand in which the metal is coordinated to O and N atoms.

In one embodiment, M may be selected from Ir and Pt.

Non-limiting examples of organic compounds that may be used as triplet host in one of the aspects of the present disclosure are selected from: compounds comprising cyclic aryl groups, such as benzene, biphenyl, triphenyl, benzo, and fluorene; compounds comprising aromatic heterocyclic groups, such as triphenylamine, dibenzothiophene, dibenzofuran, dibenzoselenophen, furan, thiophene, benzofuran, benzothiophene, benzoselenophene, carbazole, indolocarbazole, indolopyridine, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazin, oxadiazine, indole, benzimidazole, indoxazine, bisbenzoxazole, isoxazole, benzothiazole, quinoline, isoquinoline, cinnoline, quinazoline, quinoxaline, naphthalene, phthalein, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, or a combination thereof; and groups comprising 2 to 10 ring structures, which may be the same or different types of cyclic aryl or aromatic heterocyclic group and are linked to each other directly or by at least one of the following groups, such as oxygen atom, nitrogen atom, sulfur atom, silicon atom, phosphorus atom, boron atom, chain structure unit, and aliphatic ring, wherein each Ar may be further optionally substituted and the substituents may be any one of hydrogen, alkyl, alkoxy, amino, alkene, alkyne, aralkyl, heteroalkyl, aryl and heteroaryl, or a combination thereof.

In a preferred embodiment, the triplet host material may be selected from compounds comprising at least one of the following groups:

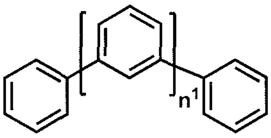
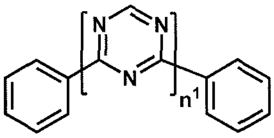
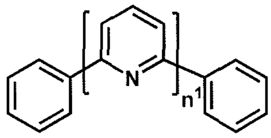

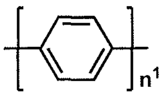
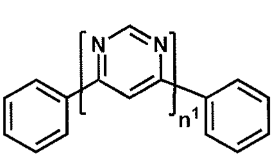
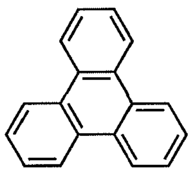
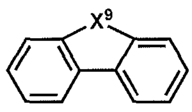
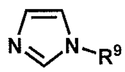

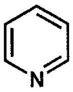
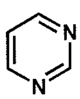
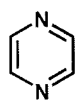
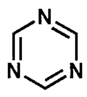
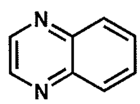
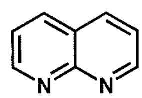
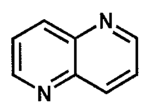

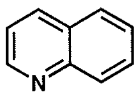
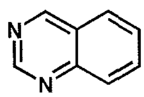
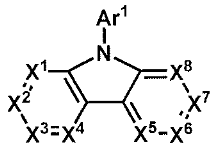
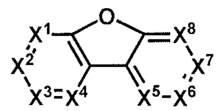
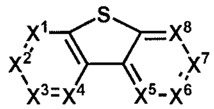

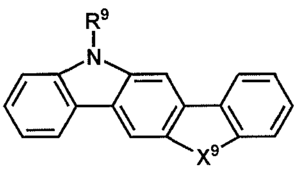
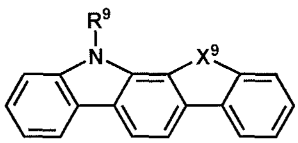
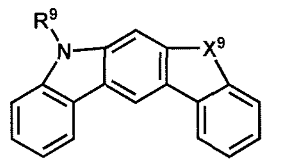

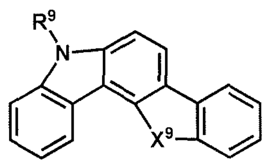
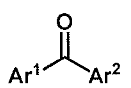
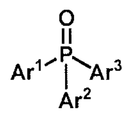
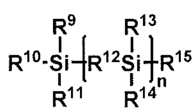

$R^9$ - $R^{15}$ each independently has the same meaning as $R^9$ above; $X^1$ - $X^8$ has the same meaning as $X^1$ - $X^8$ above; $X^9$is selected from $CR^9R^{10}$ or $NR^9$; $n^1$ can be an integer from 1 to 20.

Non-limiting examples of suitable triplet host material are listed in the following table:

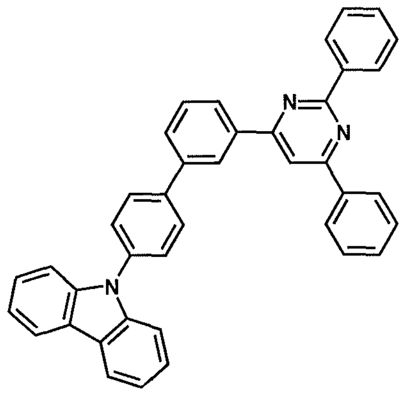
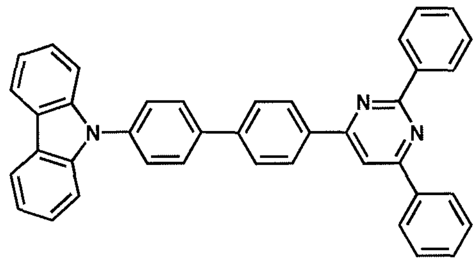

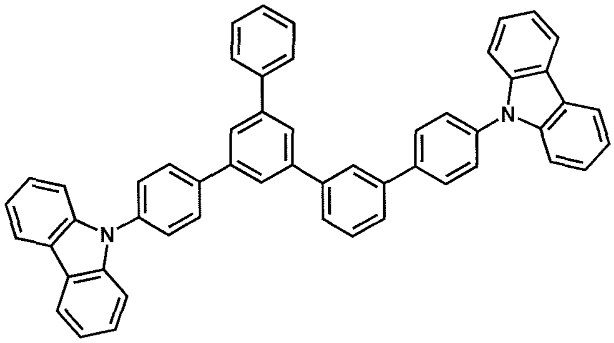
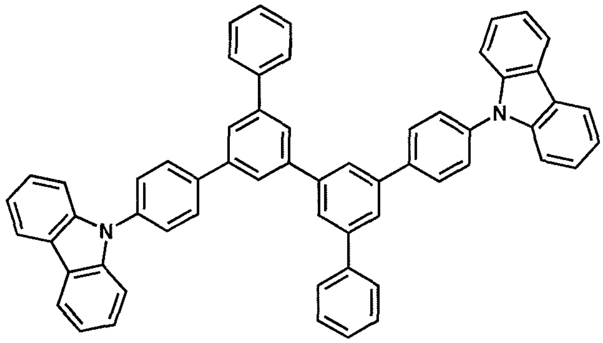

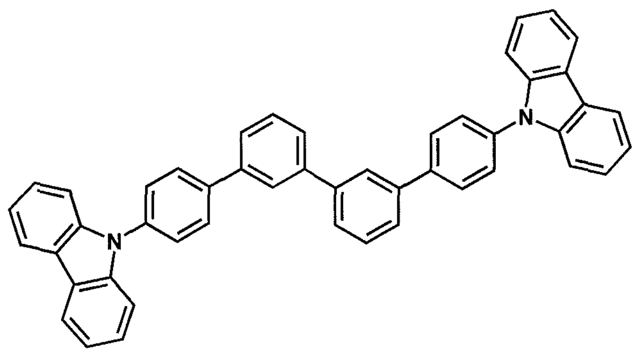

4. Singlet Host Material:

Examples of singlet host material used in one aspect of the present disclosure are not particularly limited and any organic compound may be used as the host as long as its singlet state energy is greater than that of the light emitter, especially the singlet emitter or fluorescent light emitter.

Non-limiting examples of organic compounds used as singlet host materials may be selected from: cyclic aryl compounds, such as benzene, biphenyl, triphenyl, benzo, naphthalene, anthracene, phenalene, phenanthrene, fluorene, pyrene, chrysene, perylene, azulene; aromatic heterocyclic compounds, such as triphenylamine, dibenzothiophene, dibenzofuran, dibenzoselenophen, furan, thiophene, benzofuran, benzothiophene, benzoselenophene, carbazole, indolocarbazole, indolopyridine, pyrrolodipyridine, pyrazole, imidazole, triazole, isoxazole, thiazole, oxadiazole, oxatriazole, dioxazole, thiadiazole, pyridine, pyridazine, pyrimidine, pyrazine, triazine, oxazine, oxathiazin, oxadiazine, indole, benzimidazole, indoxazine, bisbenzoxazole, isoxazole, benzothiazole, quinoline, isoquinoline, cinnoline, quinazoline, quinoxaline, naphthalene, phthalein, pteridine, xanthene, acridine, phenazine, phenothiazine, phenoxazine, benzofuropyridine, furodipyridine, benzothienopyridine, thienodipyridine, benzoselenophenopyridine, and selenophenodipyridine; and groups comprising 2 to 10 ring structures, which may be the same or different types of cyclic aryl or aromatic heterocyclic group and are linked to each other directly or by at least one of the following groups, such as oxygen atom, nitrogen atom, sulfur atom, silicon atom, phosphorus atom, boron atom, chain structure unit, and aliphatic rings.

In a preferred embodiment, the monomorphic host material may be selected from compounds comprising at least one of the following groups:

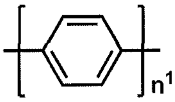 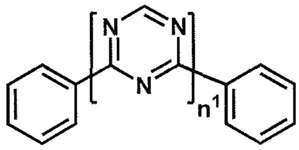 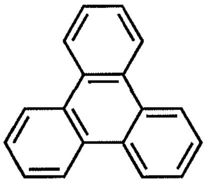 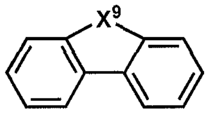 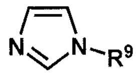

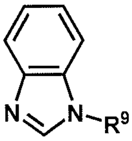 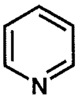 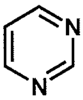 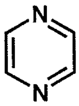 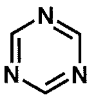 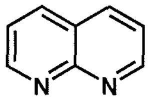 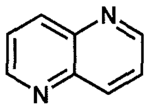

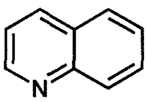 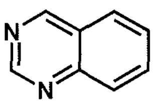 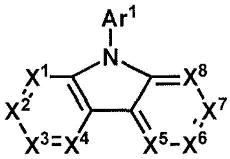 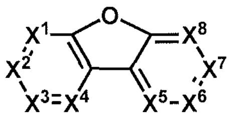 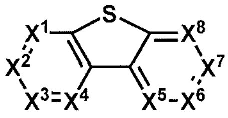

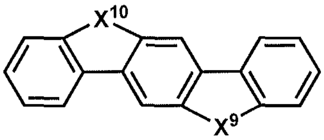 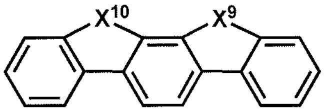 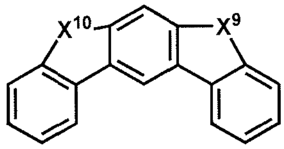

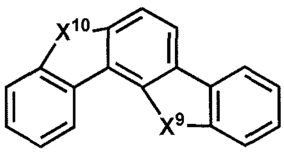 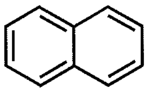 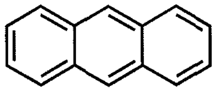 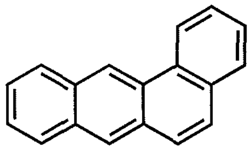

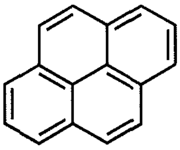 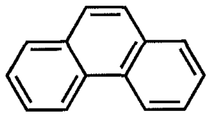 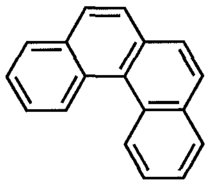

$X^{10}$ is selected from $CR^9R^{10}$ or $NR^9$; $R^9$, $Ar^1$, $X^1$ - $X^8$, $X^9$and $n^1$ have the same meaning as defined above.

Non-limiting examples of a suitable singlet host material are listed in the following table:

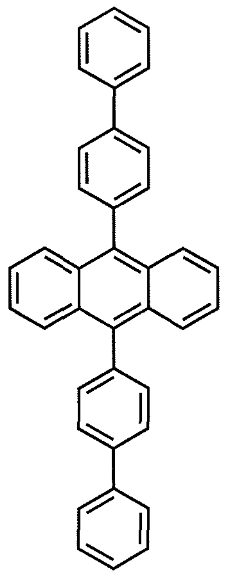

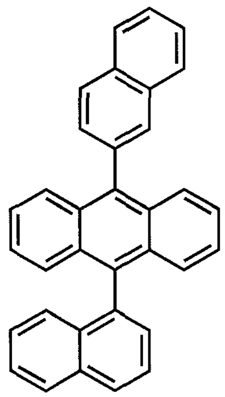

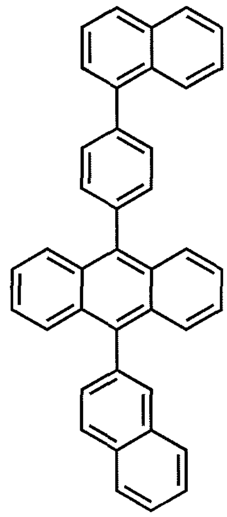

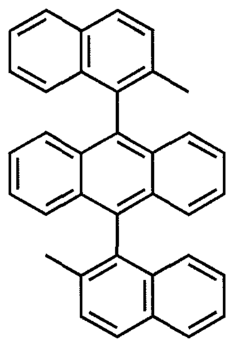

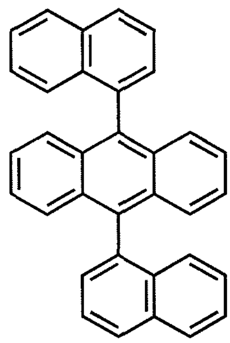

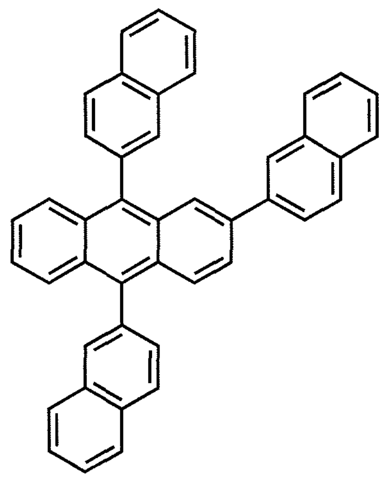

5. Singlet Emitter

The singlet emitter tends to have a longer conjugate π-electron system. To date, there have been many examples, such as, but not limited to, styrylamine and its derivatives and indenofluorene and its derivatives.

In a preferred embodiment, the singlet emitter may be selected from the group consisting of monostyrylamines, distyrylamines, tristyrylamines, tetrastyrylamines, styrylphosphines, styryl ethers, and arylamines, or combinations thereof.

Mono styrylamine refers to a compound which comprises an unsubstituted or optionally substituted styryl group and at least one amine, most preferably an aryl amine. Distyrylamine refers to a compound comprising two unsubstituted or optionally substituted styryl groups and at least one amine, most preferably an aryl amine. Ternarystyrylamine refers to a compound which comprises three unsubstituted or optionally substituted styryl groups and at least one amine, most preferably an aryl amine. Quaternarystyrylamine refers to a compound comprising four unsubstituted or optionally substituted styryl groups and at least one amine, most preferably an aryl amine. Preferred styrene is stilbene, which may be further optionally substituted. The corresponding phosphines and ethers are defined similarly to amines. Aryl amine or aryl amine refers to a compound comprising three unsubstituted or optionally substituted cyclic or heterocyclic aryl systems directly attached to nitrogen. At least one of these cyclic or heterocyclic aryl systems is preferably selected from fused ring systems and most preferably has at least 14 aromatic ring atoms. Among the preferred examples are aryl anthramine, aryl anthradiamine, aryl pyrene amines, aryl pyrene diamines, aryl chrysene amines and aryl chrysene diamine. Aryl anthramine refers to a compound in which a diarylamino group is directly attached to anthracene, most preferably at position 9. Aryl anthradiamine refers to a compound in which two diarylamino groups are directly attached to anthracene, most preferably at positions 9, 10. Aryl pyrene amines, aryl pyrene diamines, aryl chrysene amines and

aryl chrysene diamine are similarly defined, wherein the diarylarylamino group is most preferably attached to position 1 or 1 and 6 of pyrene.

Non-limiting examples of singlet emitter based on vinylamine and arylamine are also preferred examples which may be found in the following patent documents: WO 2006/000388, WO 2006/058737, WO 2006/000389, WO 2007/065549 , WO 2007/115610, US 7250532 B2, DE 102005058557 A1, CN 1583691 A, JP 08053397 A, US 6251531 B1, US 2006/210830 A, EP 1957606 A1, and US 2008/0113101 A1.

Non-limiting examples of singlet light emitters based on distyrylbenzene and its derivatives may be found in, for example, US 5121029.

Further preferred singlet emitters may be selected from the group consisting of: indenofluorene-amine and indenofluorene-diamine as disclosed in WO 2006/122630, benzoindenofluorene-amine and benzoindenofluorene-diamine as disclosed in WO 2008/006449, dibenzoindenofluorene-amine and dibenzoindenofluorene-diamine as disclosed in WO2007/140847.

Other materials useful as singlet emitters include polycyclic aryl compounds, especially any one selected from the derivatives of the following compounds: anthracenes such as 9,10-di -naphthylanthracene, naphthalene, tetraphenyl, phenanthrene, perylene such as 2,5,8,11-tetra-t-butylatedylene, indenoperylene, phenylenes such as 4,4'-(bis (9-ethyl-3-carbazovinylene) -1,1'-biphenyl, periflanthene, decacyclene, coronene, fluorene, spirobifluorene, arylpyren (e.g., US20060222886), arylenevinylene (e.g., US5121029, US5130603), cyclopentadiene such as tetraphenylcyclopentadiene, rubrene, coumarine, rhodamine, quinacridone, pyrane such as 4 (dicyanoethylene) -6-(4-dimethylaminostyryl-2-methyl) -4H-pyrane (DCM), thiapyran, bis (azinyl) imine-boron compounds (US 2007/0092753 A1), bis (azinyl) methene compounds, carbostyryl compounds, oxazone, benzoxazole, benzothiazole, benzimidazole, and diketopyrrolopyrrole, or combinations thereof. Non-limiting examples of some singlet emitter material may be found in the following patent documents: US 20070252517 A1, US 4769292, US 6020078, US 2007/0252517 A1, and US 2007/0252517 A1.

Non-limiting examples of suitable singlet emitters are listed in the following table:

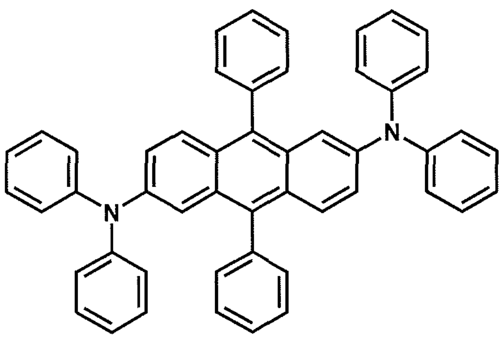

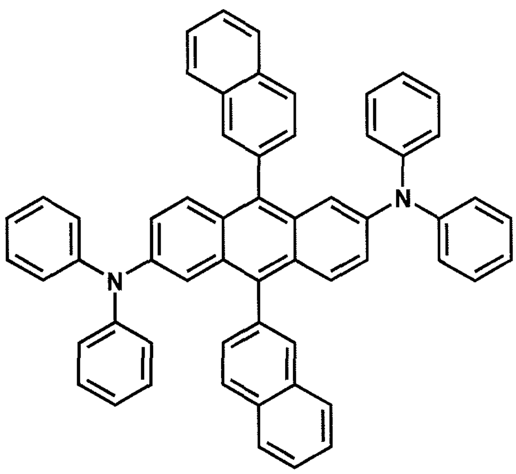

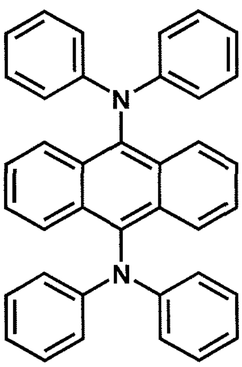

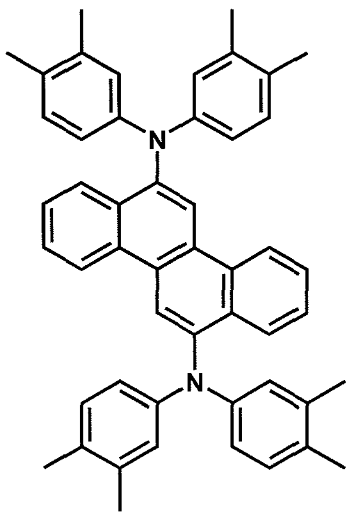

6. Triplet Emitter

The triplet emitter used in one aspect of the present disclosure is also called a phosphorescent emitter. In a preferred embodiment, the triplet emitter may be a metal complex of the general formula M (L) n, wherein M may be a metal atom; L may be a same or different ligand each time it is present, and may be bonded or coordinated to the metal atom M at one or more positions; n may be an integer greater than 1, preferably 1, 2, 3, 4, 5 or 6. Alternatively, these metal complexes may be attached to a polymer by one or more positions, most preferably through an organic ligand.

In a preferred embodiment, the metal atom M may be selected from the group consisting of transition metal elements or lanthanides or actinides, preferably Ir, Pt, Pd, Au, Rh, Ru, Os, Sm, Eu, Gd, Tb, Dy , Re, Cu or Ag, and particularly preferably Os, Ir, Ru, Rh, Re, Pd, or Pt.

Preferably, the triplet emitter comprises a chelating ligand, i.e., a ligand, coordinated to the metal by at least two bonding sites, and it is particularly preferred that the triplet emitter comprises two or three identical or different bidentate or multidentate ligand. Chelating ligands help to improve stability of metal complexes.

Non-limiting examples of organic ligands may be selected from the group consisting of phenylpyridine derivatives, 7,8-benzoquinoline derivatives, 2 (2 -thienyl) pyridine derivatives, 2 (1-naphthyl) pyridine derivatives, or 2 phenylquinoline derivatives. All of these organic ligands may be optionally substituted, for example, optionally substituted with fluoromethyl or trifluoromethyl. The auxiliary ligand may be preferably selected from acetylacetonate or picric acid.

In a preferred embodiment, the metal complex which may be used as the triplet emitter may have the following form:

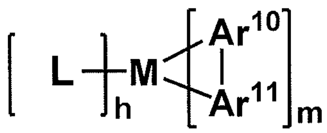

wherein M is a metal selected from the group consisting of transition metal elements or lanthanides or actinides; $Ar^{10}$ may be the same or different cyclic group each time it is present, which comprises at least one donor atom, that is, an atom with a lone pair of electrons, such as nitrogen atom or phosphorus atom, which is coordinated to the metal through its ring group; $Ar^{11}$ may be the same or different cyclic group comprising at least one C atom and is coordinated to the metal through its ring group; $Ar^{10}$ and $Ar^{11}$ are covalently bonded together, wherein each of them may carry one or more substituents which may also be joined together by substituents; L may be the same or different at each occurrence and is an auxiliary ligand, preferably a bidentate chelating ligand, and most preferably a monoanionic bidentate chelating ligand; m is 1, 2 or 3, preferably 2 or 3, and particularly preferably 3; and, N is 0, 1, or 2, preferably 0 or 1, particularly preferably 0.

Non-limiting examples of triplet emitter materials that are extremely useful may be found in the following patent documents and references: WO 200070655, WO 200141512, WO 200202714, WO 200215645, EP 1191613, EP 1191612, EP 1191614, WO 2005033244, WO 2005019373, US 2005/0258742, WO 2009146770, WO 2010015307, WO 2010031485, WO 2010054731, WO 2010054728, WO 2010086089, WO 2010099852, WO 2010102709, US 20070087219 A1, US 20090061681 A1, US 20010053462 A1, Baldo, Thompson et al. Nature 403, (2000), 750-753, US 20090061681 A1, US 20090061681 A1, Adachi et al. Appl. Phys. Lett. 78 (2001), 1622-1624, J. Kido et al. Appl. Phys. Lett. 65 (1994), 2124, Kido et al. Chem. Lett. 657, 1990, US 2007/0252517 A1, Johnson et al., JACS 105, 1983, 1795, Wrighton, JACS 96, 1974, 998, Ma et al., Synth. Metals 94, 1998, 245, US 6824895, US 7029766, US 6835469, US 6830828, US 20010053462 A1, WO 2007095118 A1, US 2012004407A1, WO 2012007088A1, WO2012007087A1, WO 2012007086A1, US 2008027220A1, WO 2011157339A1, CN 102282150A, WO 2009118087A1.

Non-limiting examples of suitable triplet emitter are given in the following table:

| | | |
|---|---|---|
| 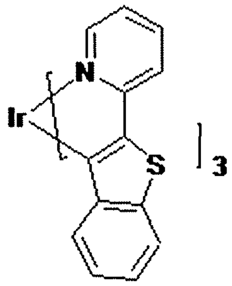 | 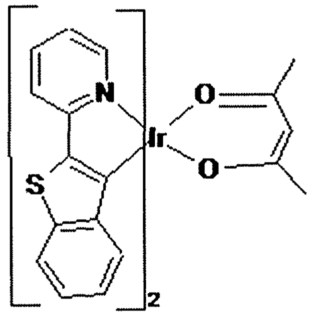 | 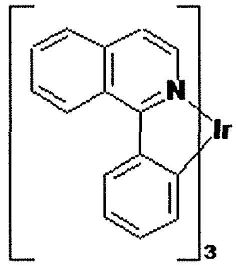 |
| 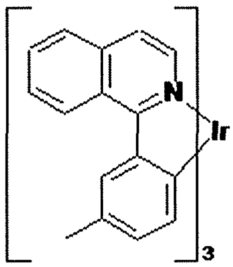 | 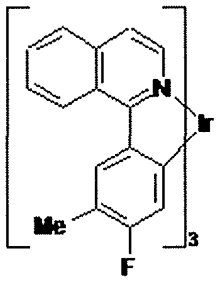 | 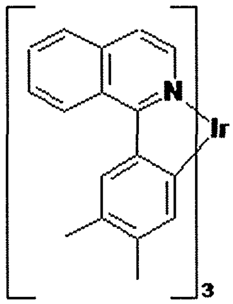 |
| 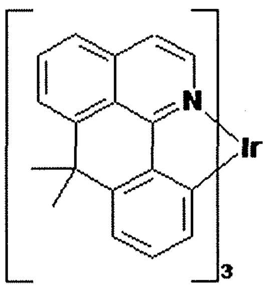 | 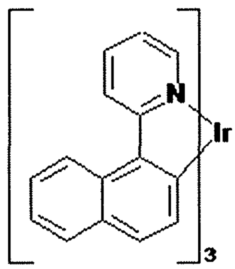 | 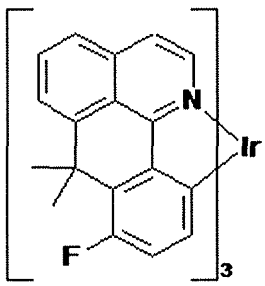 |
| 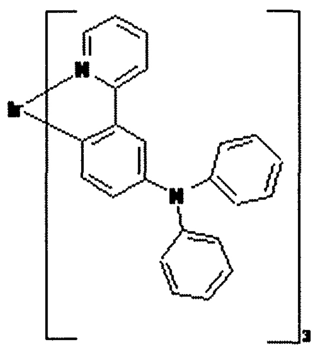 | 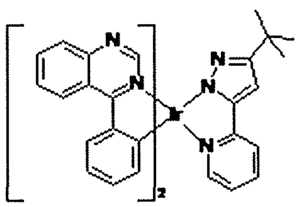 | 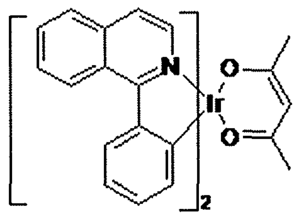 |

| | | |
|---|---|---|
| 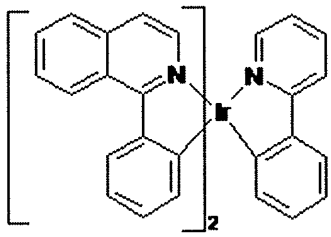 | 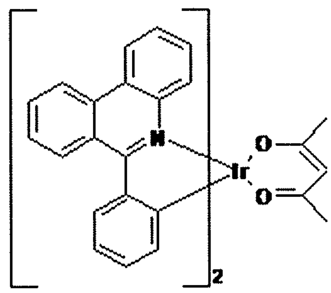 | 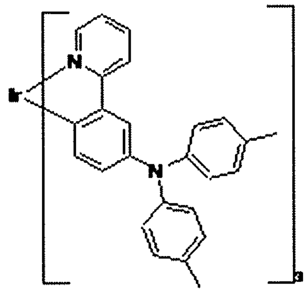 |
| 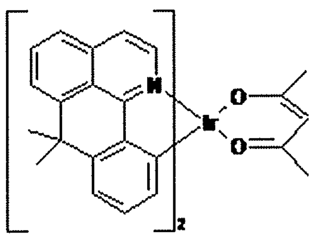 | 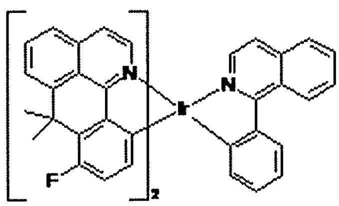 | 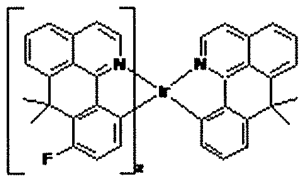 |
| 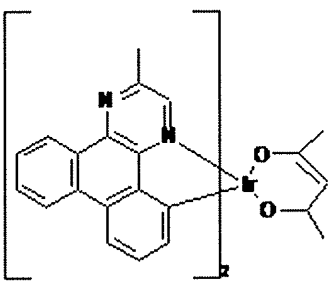 | 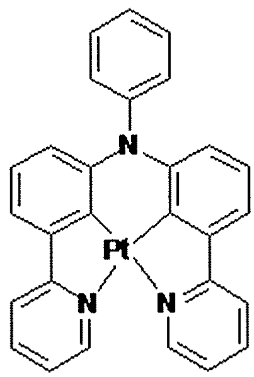 | 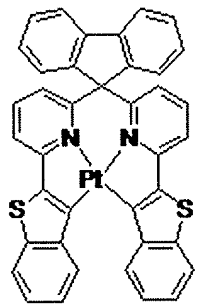 |
| 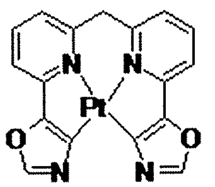 | 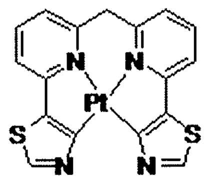 | 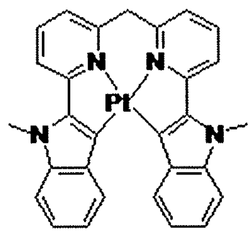 |
| 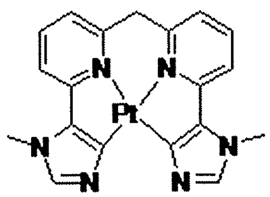 | 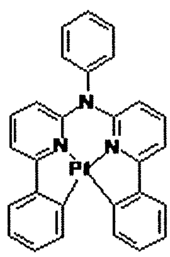 | 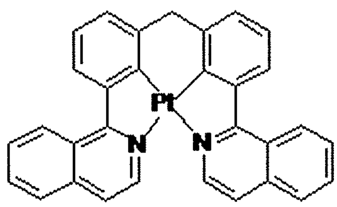 |

| | | |
|---|---|---|
| 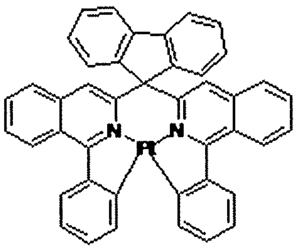 | 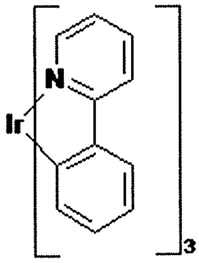 | 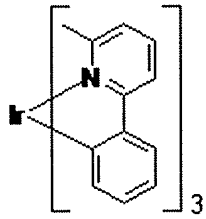 |
| 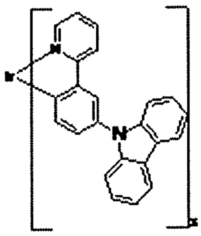 | 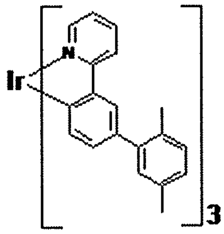 | 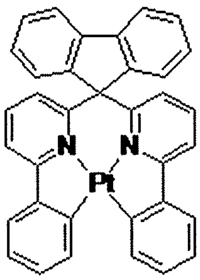 |
| 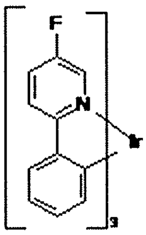 | 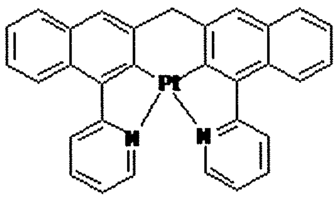 | 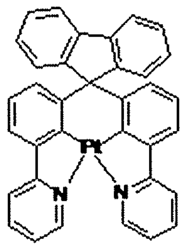 |
| 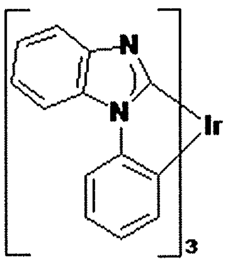 | 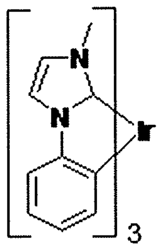 | 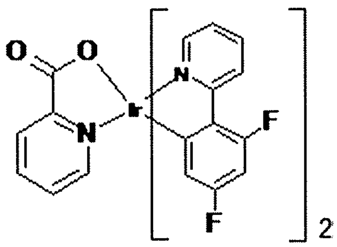 |
| 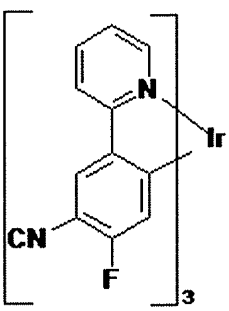 | 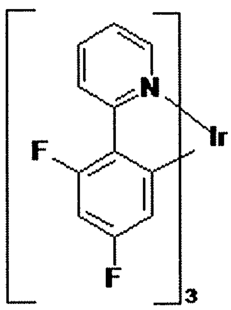 | 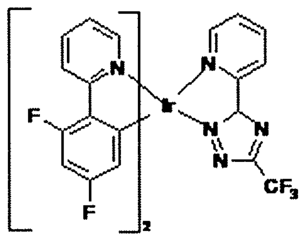 |

| | | |
|---|---|---|
| 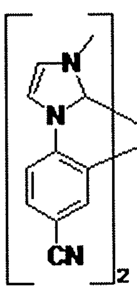 | 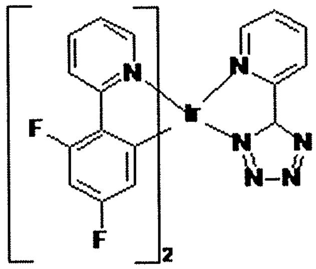 | 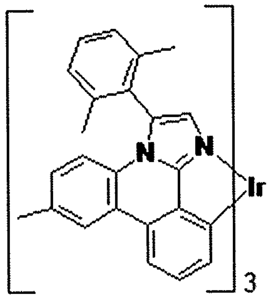 |
| 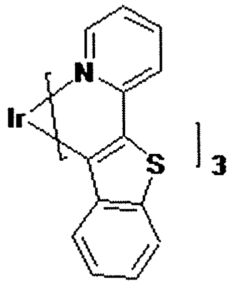 | 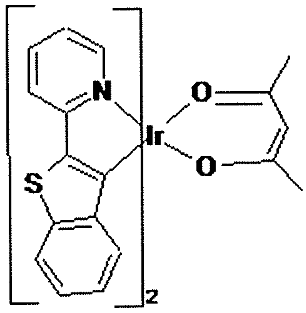 | 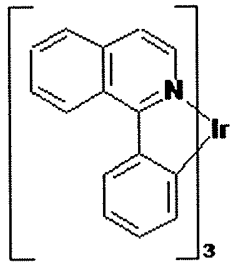 |
| 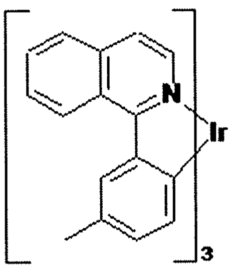 | 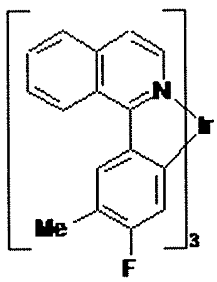 | 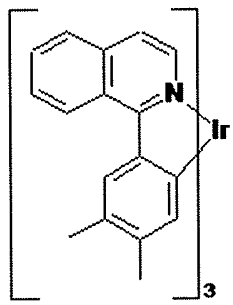 |
| 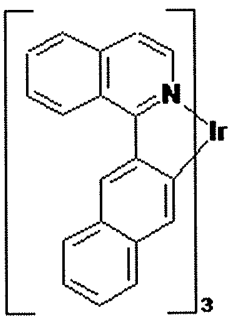 | 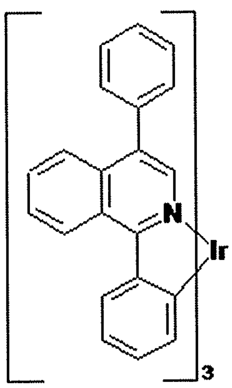 | 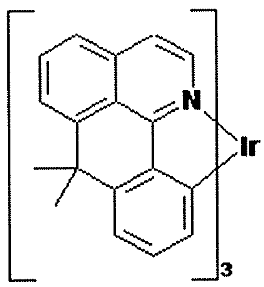 |

| | | |
|---|---|---|
| 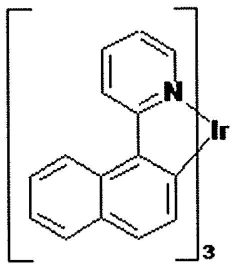 | 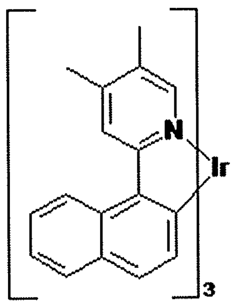 | 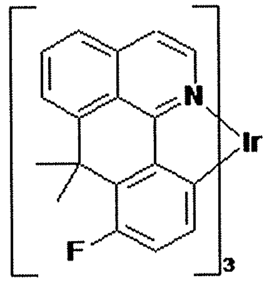 |
| 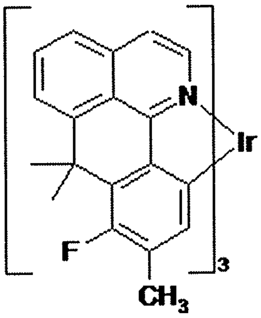 | 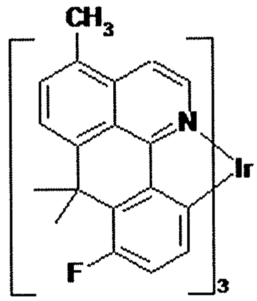 | 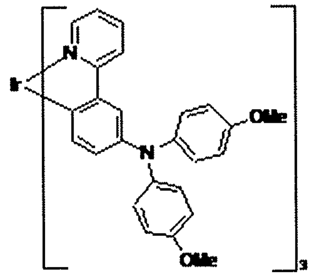 |
| 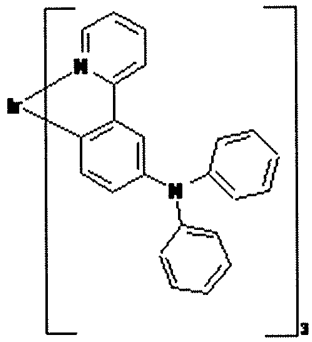 | 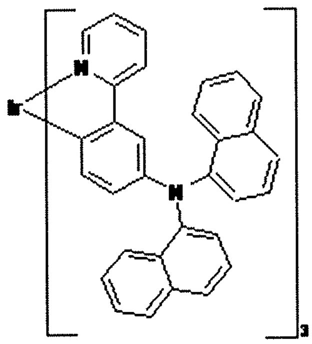 | 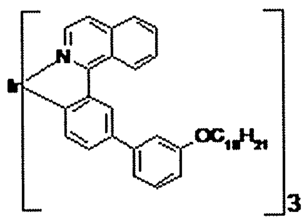 |
| 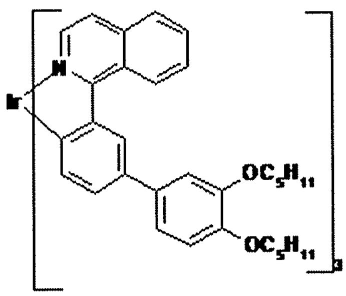 | 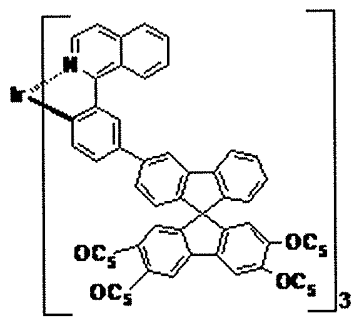 | 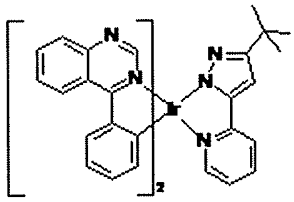 |

| | | |
|---|---|---|
| 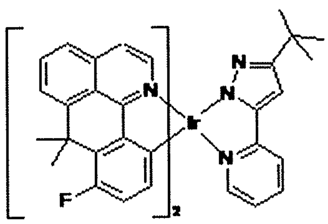 | 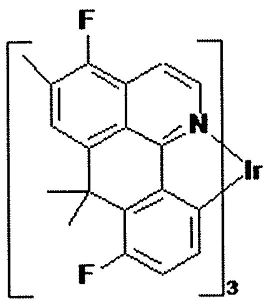 | 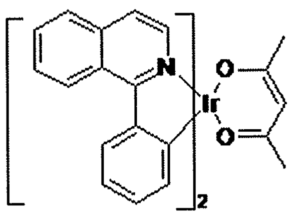 |
| 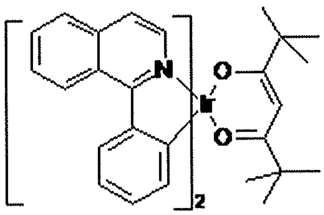 | 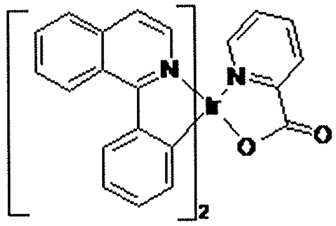 | 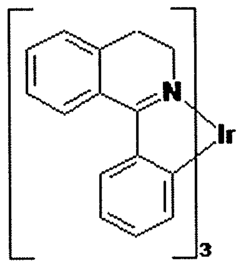 |
| 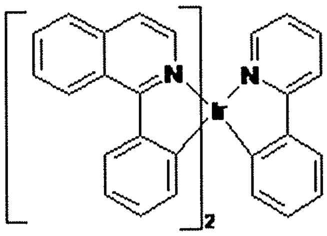 | 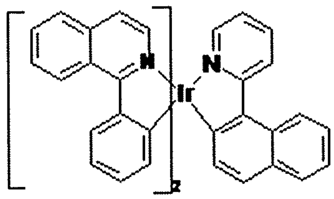 | 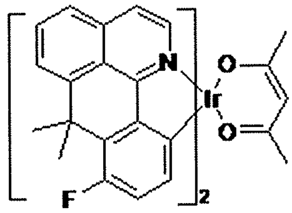 |
| 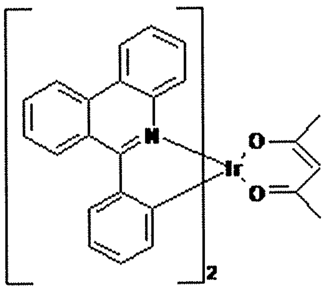 | 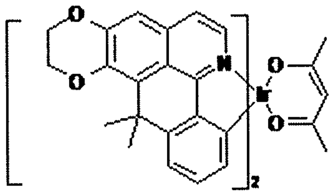 | 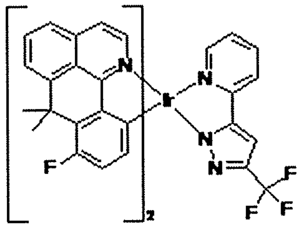 |
| 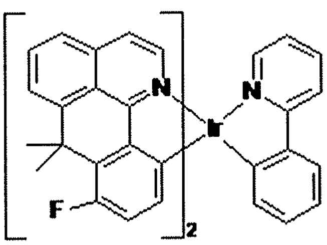 | 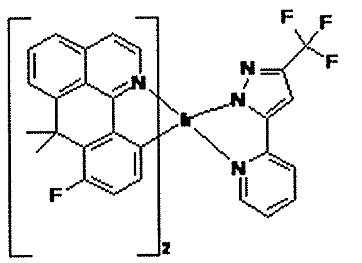 | 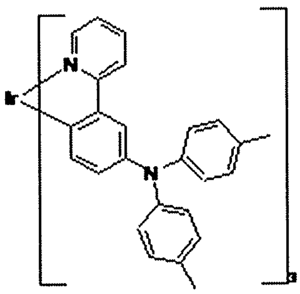 |

| | | |
|---|---|---|
| 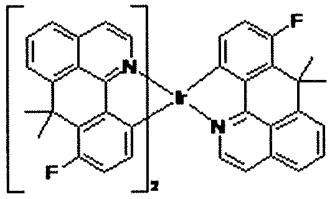 | 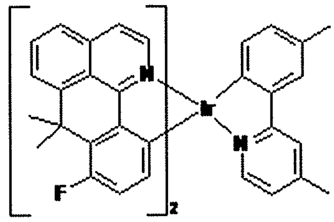 | 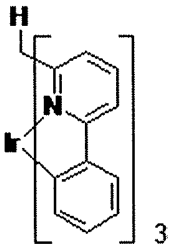 |
| 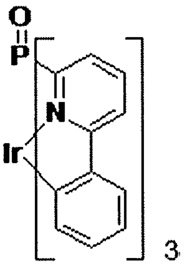 | 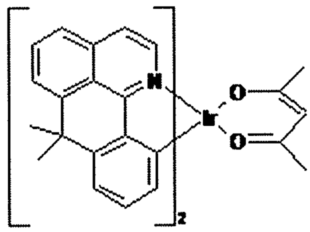 | 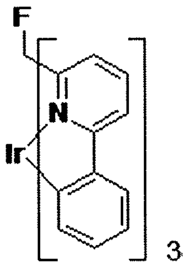 |
| 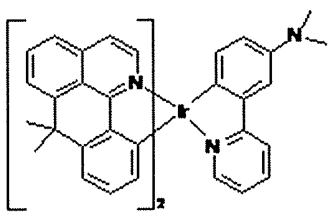 | 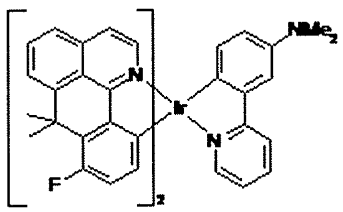 | 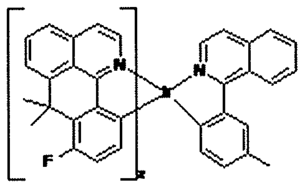 |
| 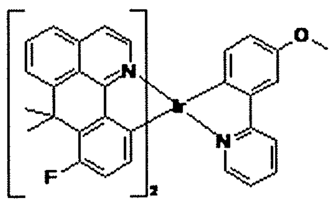 | 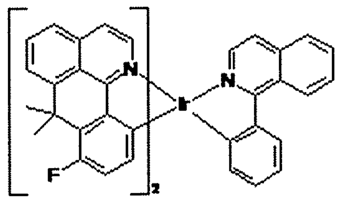 | 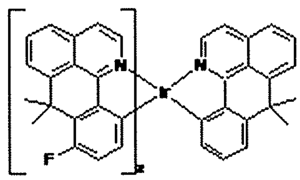 |
| 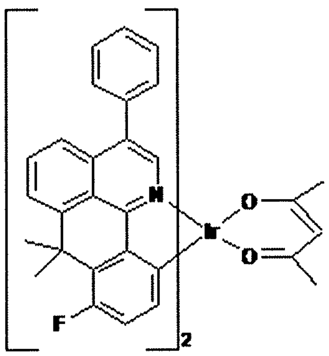 | 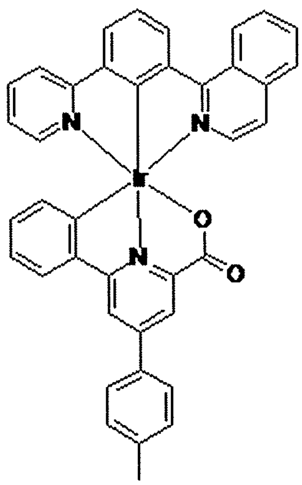 | 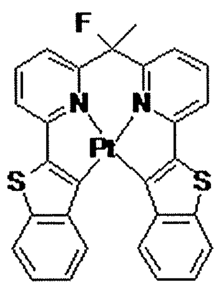 |

| | | |
|---|---|---|
| 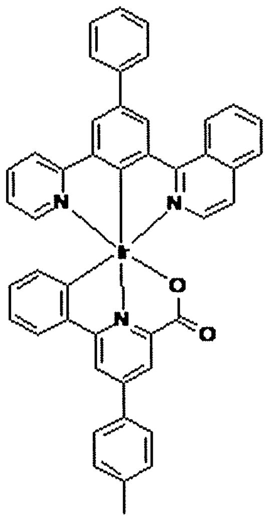 | 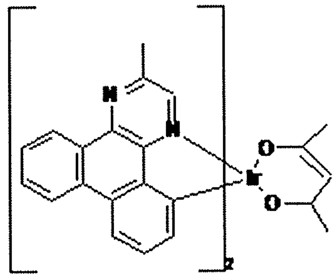 | 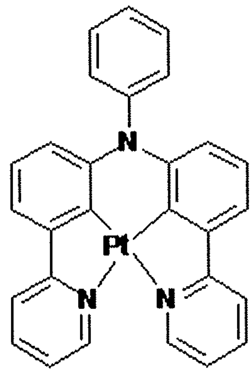 |
| 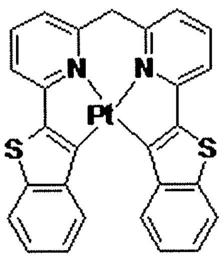 | 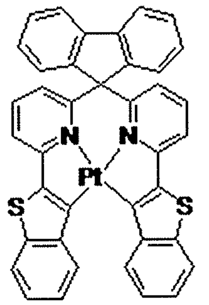 | 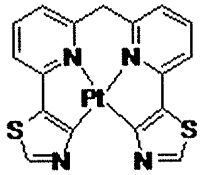 |
| 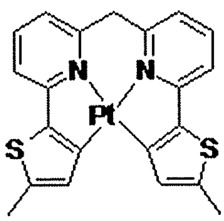 | 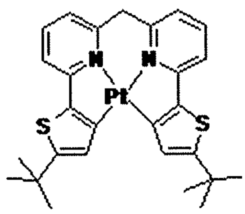 | 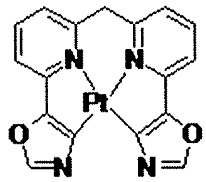 |
| 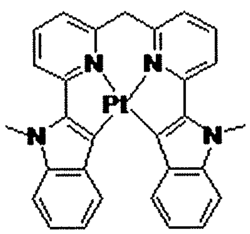 | 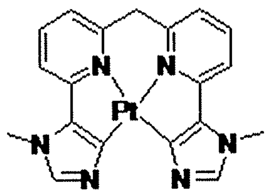 | 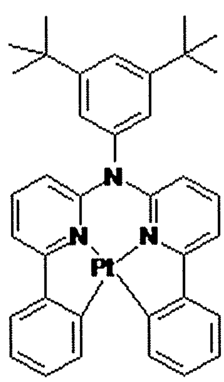 |

| | | |
|---|---|---|
| 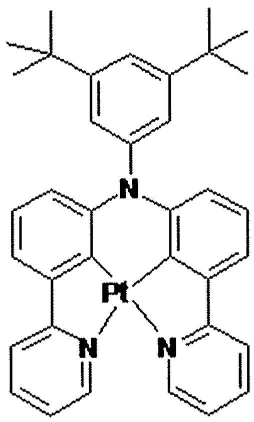 | 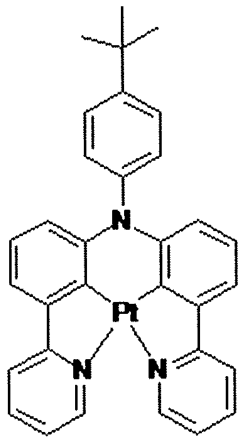 | 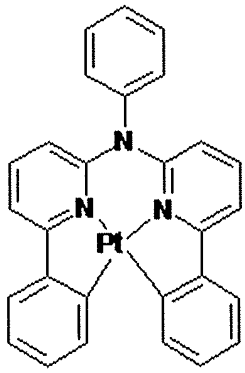 |
| 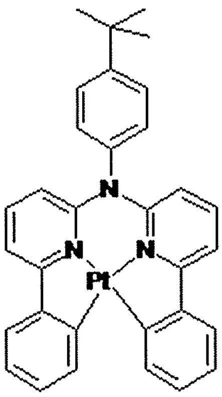 | 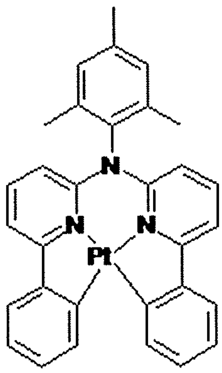 | 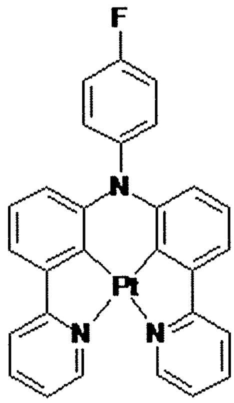 |
| 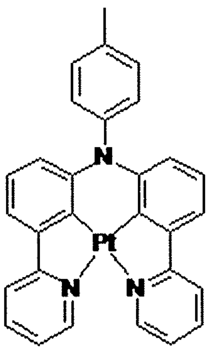 | 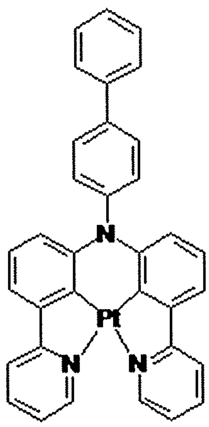 | 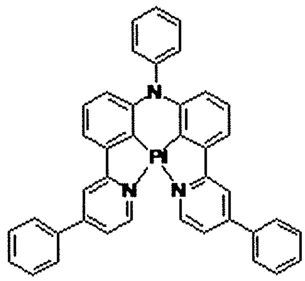 |

| | | |
|---|---|---|
| 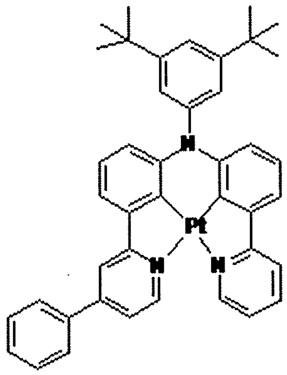 | 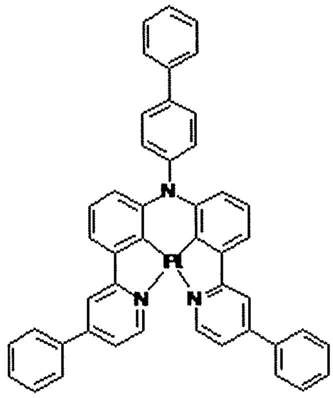 | 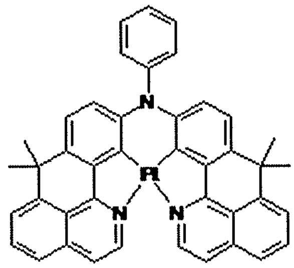 |
| 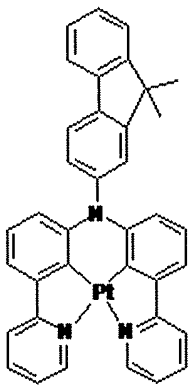 | 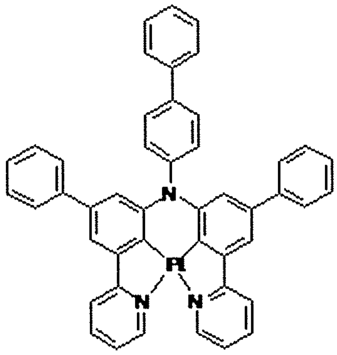 | 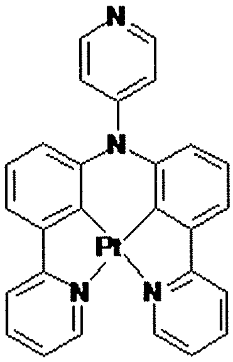 |
| 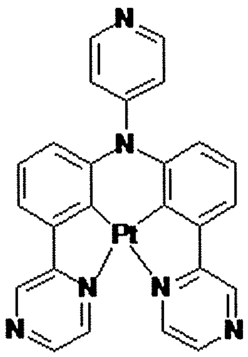 | 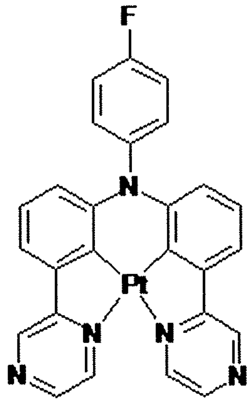 | 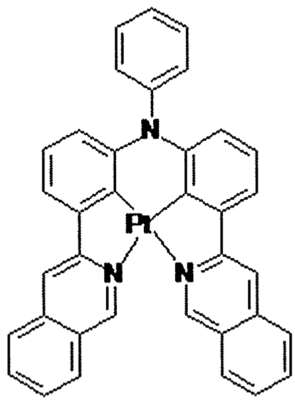 |
| 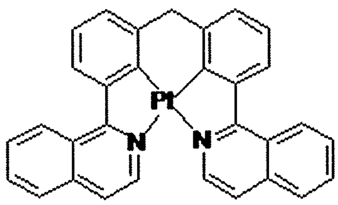 | 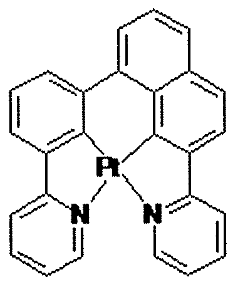 | 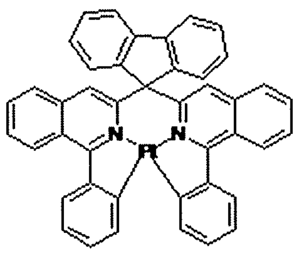 |

| | | |
|---|---|---|
| 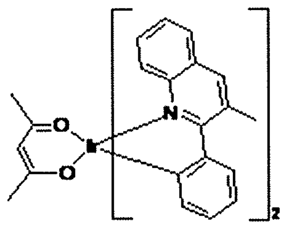 | 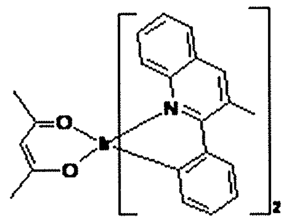 | 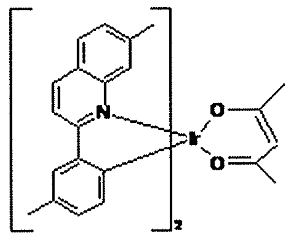 |
| 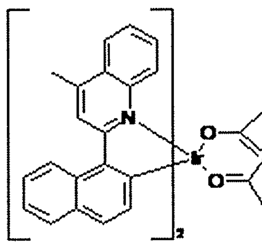 | 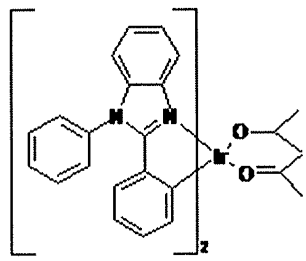 | 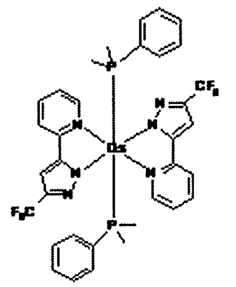 |
| 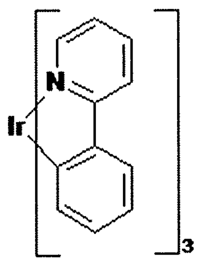 | 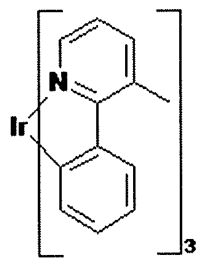 | 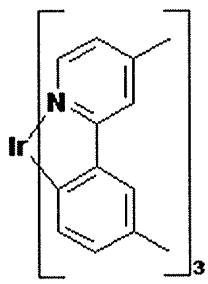 |
| 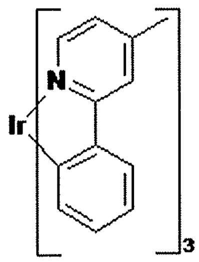 | 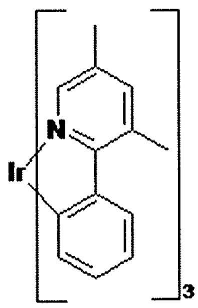 | 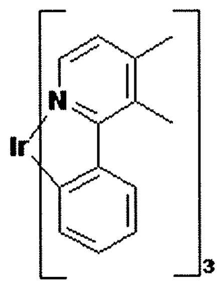 |
| 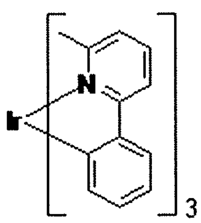 | 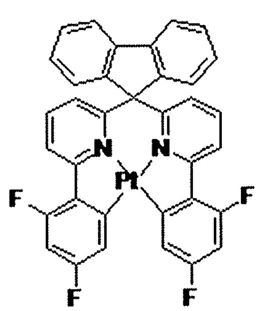 | 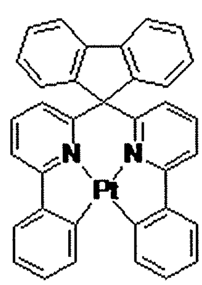 |

| | | |
|---|---|---|
| 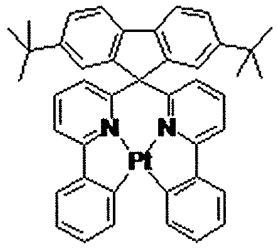 | 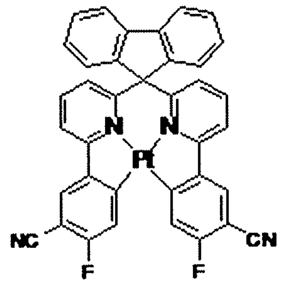 | 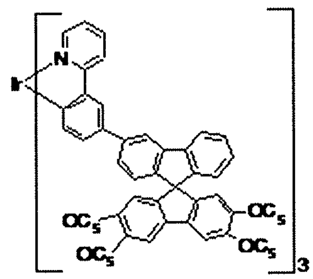 |
| 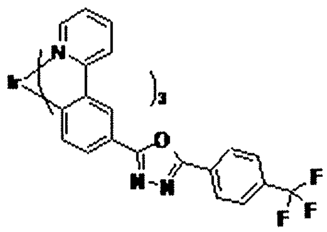 | 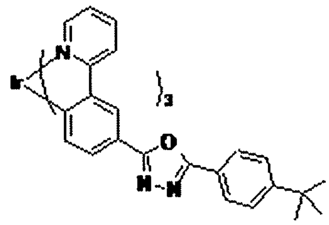 | 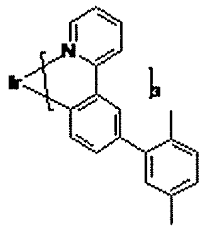 |
| 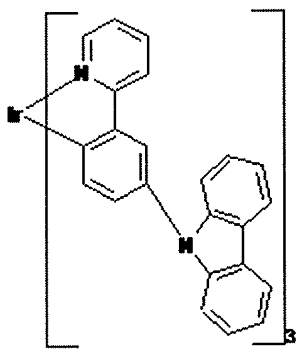 | 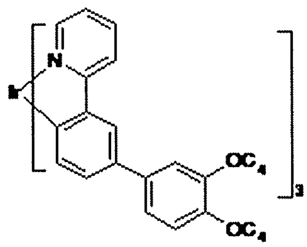 | 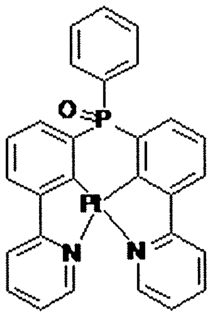 |
| 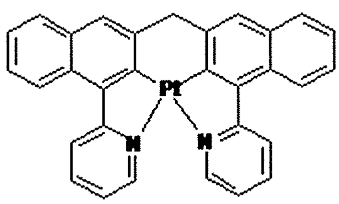 | 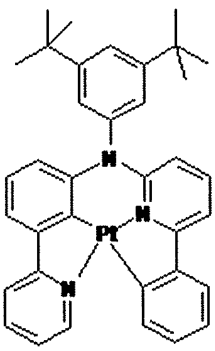 | 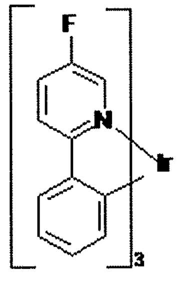 |

| | | |
|---|---|---|
| 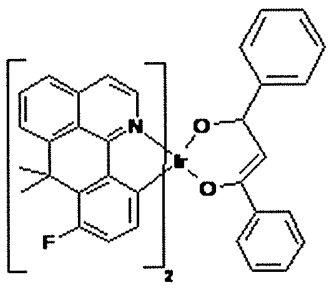 | 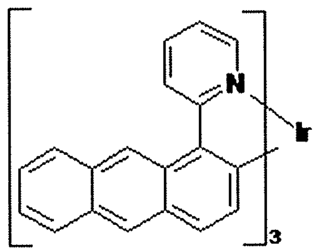 | 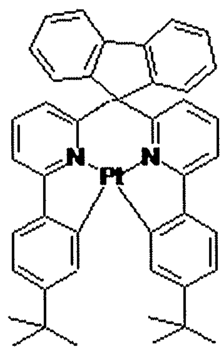 |
| 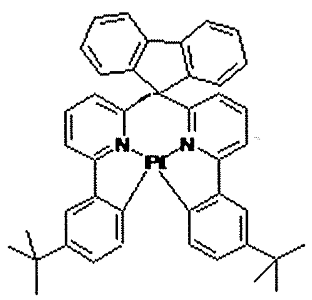 | 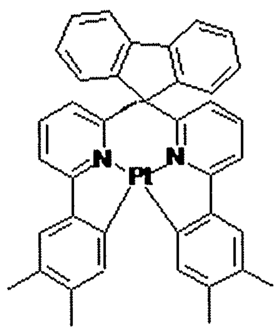 | 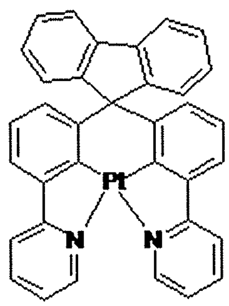 |
| 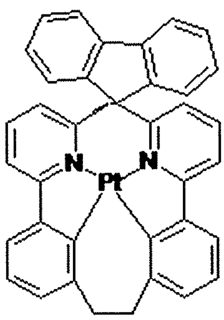 | 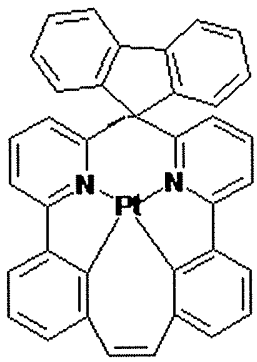 | 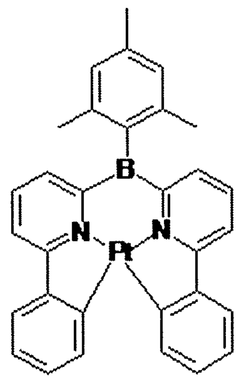 |
| 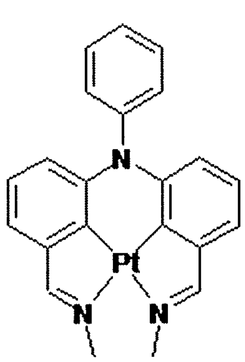 | 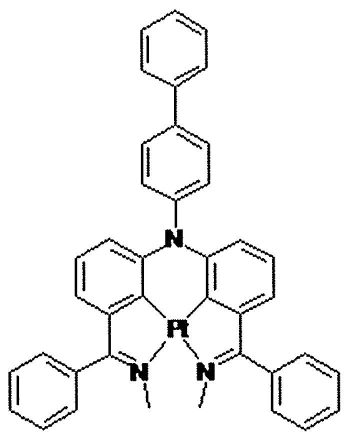 | 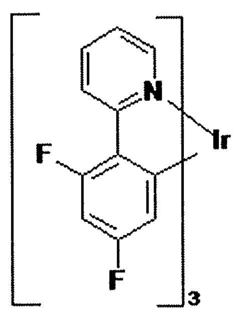 |

| | | |
|---|---|---|
| 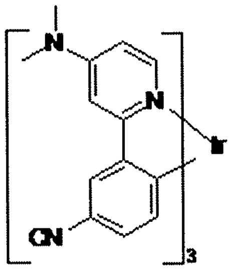 | 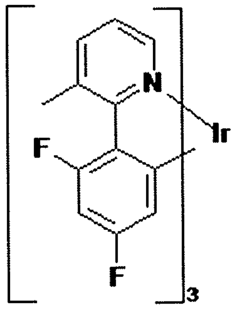 | 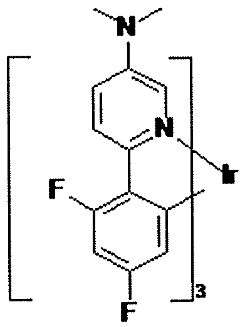 |
| 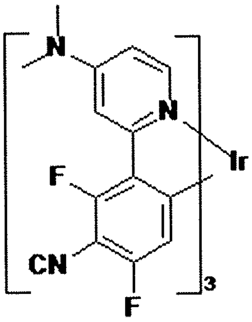 | 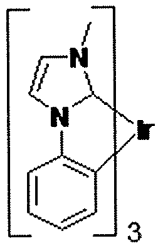 | 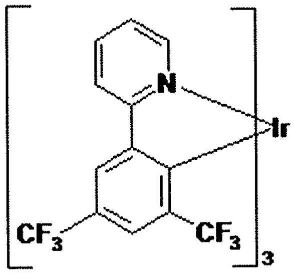 |
| 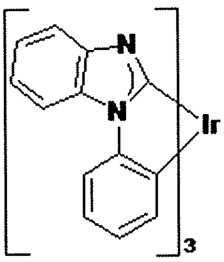 | 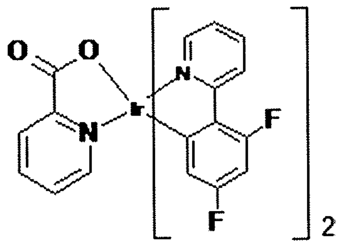 | 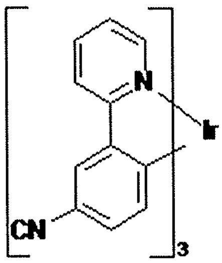 |
| 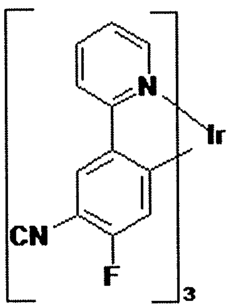 | 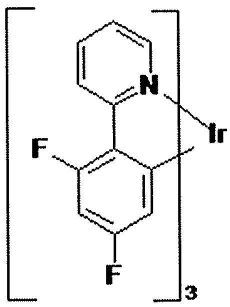 | 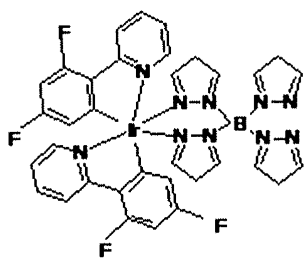 |

| | | |
|---|---|---|
| 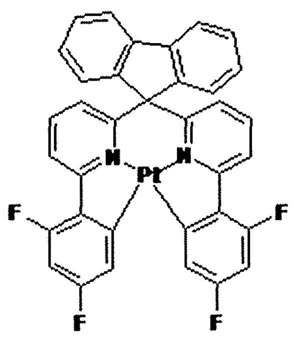 | 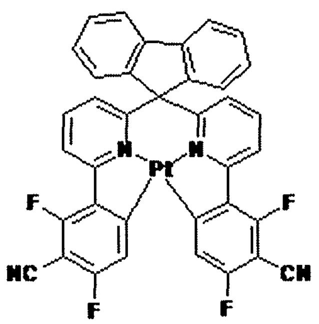 | 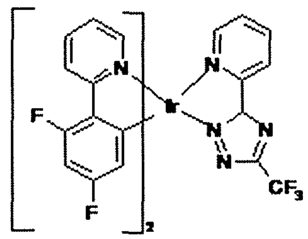 |
| 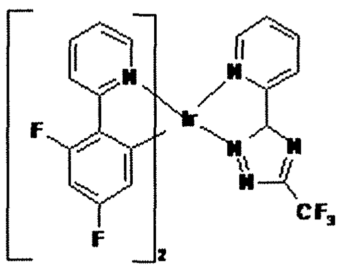 | 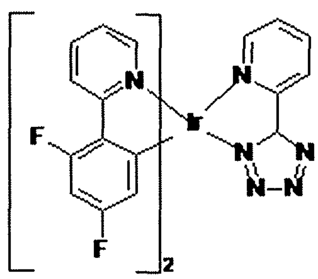 | 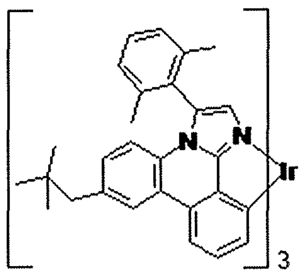 |
| 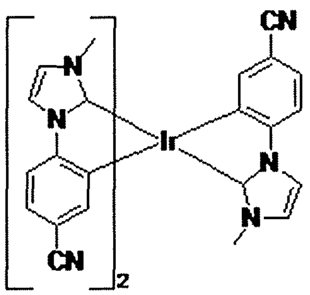 | 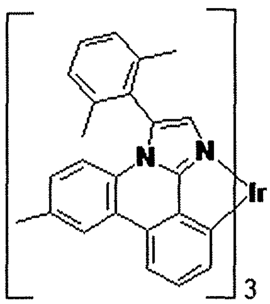 | |

7. Polymers

In some embodiments, the organic functional materials described above, including HIM, HTM, ETM, EIM, Host, fluorescent emitter, and phosphorescent emitters, may be in the form of polymers.

In a preferred embodiment, the polymer suitable for the present disclosure is a conjugated polymer. In general, the conjugated polymer may have the general formula:

$$-\left[B\right]_x\left[A\right]_y-$$

Chemical Formula 1

wherein B, A may be independently selected as the same or different structural elements in multiple occurrences

B: a π-conjugated structural unit with relatively large energy gap, also referred to as backbone unit, which may be selected from monocyclic or polycyclic aryl or heteroaryl, preferably in the form of benzene, biphenylene, naphthalene, anthracene, phenanthrene, dihydrophenanthrene, 9,10-dihydrophenanthroline, fluorene, difluorene, spirobifluorene, p-phenylenevinylene, trans-indenofluorene, cis-indenofluorene, dibenzol-indenofluorene, indenonaphthalene and derivatives thereof.

A: a $\pi$-conjugated structural unit with relatively small energy gap, also referred to as a functional unit, which, according to different functional requirements, may be selected from the above-mentioned hole-injection or hole-transport material (HIM/HTM), hole-blocking material (HBM), electron-injection or electron-transport material (EIM/ETM), electron-blocking material (EBM), organic host material (Host), singlet emitter (fluorescent emitter), multiplet emitter (phosphorescent emitter).

$$x, y: > 0, \text{ and } x + y = 1.$$

In a preferred embodiment, the polymeric HTM material may be a homopolymer, wherein preferred homopolymers may be selected from the group consisting of polythiophene, polypyrrole, polyaniline, polybiphenyl triarylamine, polyvinylcarbazole and their derivatives.

In another preferred embodiment, the polymeric HTM material may be a conjugated copolymer represented by Chemical Formula 1, wherein

A: a functional group having a hole-transport capacity, which may be selected from structural units comprising the above-mentioned hole-injection or hole-transport material (HIM/HTM); in a preferred embodiment, A may be selected from the group consisting of amines, biphenyl triarylamine, thiophene, thiophthene such as dithienothiophthene and thiophthene, pyrrole, aniline, carbazole, indenocarbazole, indolocarbazole, pentacene, phthalocyanine, porphyrins and their derivatives.

x, y: >0, and x + y = 1; typically y≥0.10, more preferably y ≥0.15, more preferably y≥ 0.20, and most preferably x = y = 0.5.

Non-limiting examples of suitable HTM compounds are listed in the following table:

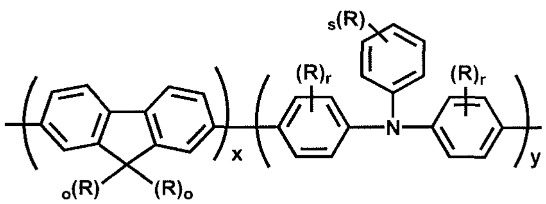

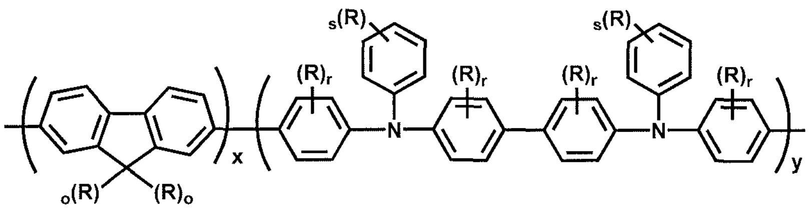

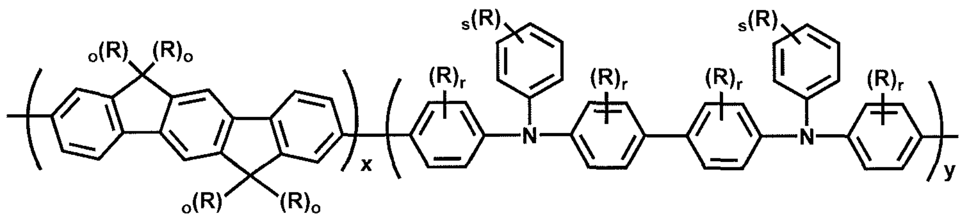

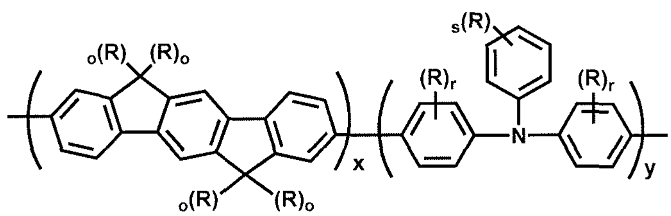

wherein

$R_o$, $R_r$ and $R_s$ may each independently be: linear alkyl, alkoxy or thioalkoxy having 1 to 20 C atoms; or branched or cyclic alkyl, alkoxy, thioalkoxy or silyl having 3 to 20 C atoms; or optionally substituted keto having 1 to 20 C atoms, alkoxycarbonyl having 2 to 20 C atoms, aryloxycarbonyl having 7 to 20 C atoms, cyano (-CN), carbamoyl (-C(=O)$NH_2$), haloformyl (-C (= O) -X, wherein X represents a halogen atom), formyl groups (-C (= O) -H), isocyanato, isocyanate groups, thiocyanate groups or isothiocyanates, hydroxyl, nitro, $CF_3$, Cl, Br, F, crosslinkable group or optionally substituted or unsubstituted aryl or heterocyclic aryl having 5 to 40 ring atoms, or aryloxy or heteroaryloxy having 5 to 40 ring atoms, or a combination of these systems;

x, y: >0, and x + y = 1; typically y≥0.10, more preferably y ≥0.15, more preferably y≥ 0.20, and most preferably x = y = 0.5.

Another preferred type of organic ETM material is a polymer having an electron-transport capacity, including conjugated polymer and nonconjugated polymer.

Preferred polymeric ETM materials are homopolymers, and preferred homopolymers are selected from the group consisting of polyphenanthrene, polyphenanthroline, polyindenofluorene, polyspirobifluorene, polyfluorene and their derivatives.

The preferred polymer ETM material is a conjugated copolymer represented by Chemical Formula 1, wherein A may be independently selected from the same or different forms in multiple occurrences:

A: a functional group having an electron-transport capacity, preferably selected from the group consisting of tris (8-hydroxyquinoline) aluminum ($AlQ_3$), benzene, diphenylene, naphthalene, anthracene, phenanthrene, dihydrophenanthrene, fluorene, difluorene, spirobifluorene , p-phenyleneacetylene, pyrene, perylene, 9,10-dihydrophenanthrene, phenazine, phenanthroline, trans-indenofluorene, cis-indene, dibenzo-indenofluorene, indenaphthalene, benzanthracene and their derivatives.

x, y: >0, and x + y = 1; typically y≥0.10, more preferably y ≥0.15, more preferably y≥ 0.20, and most preferably x = y = 0.5.

In a preferred embodiment, the luminescent polymer may be a conjugated polymer polymer of the general formula below:

$$-\left[B\right]_x-\left[A_1\right]_y-\left[A_2\right]_z-$$

Chemical formula 2

B: having the saming meaning as defined in Chemical Formula 1.

$A_1$: a functional group having a hole-transport or electron-transport ability, which may be selected from structural units comprising the above-mentioned hole-injection or hole-transport material (HIM/HTM), or electron-injection or electron-transport material (EIM/ETM).

$A_2$: a group having a light-emitting function, which may be selected from a structural unit comprising a singlet emitter (fluorescent light emitter) or a triplet light emitter (phosphorescent light emitter) described above.

$$x, y, z:> 0, \text{ and } x + y + z = 1.$$

In another embodiment, the polymers suitable for the present disclosure may be non-conjugated polymers. The nonconjugated polymer may be the backbone with all functional groups on the side chain. Non-limiting examples of such nonconjugated polymers for use as phosphorescent host or phosphorescent emitter materials may be found in patent applications such as US 7250226 B2, JP2007059939A, JP2007211243A2 and JP2007197574A2. Non-limiting examples of such nonconjugated polymers used as fluorescent light-emitting materials may be found in the patent applications JP2005108556, JP2005285661, and JP2003338375. In addition, the non-conjugated polymer may also be

a polymer, with the conjugated functional units on the backbone linked by non-conjugated linking units. Non-limiting examples of such polymers are disclosed in DE102009023154.4 and DE102009023156.0.

In some embodiments, in a mixture according to one aspect of the present disclosure, the organic compound described in one aspect of the present disclosure may be present in an amount of from 0.01 to 90 wt%, more preferably from 1 to 80 wt%, even more preferably 10 to 80 wt%, and most preferably 20 to 70 wt%.

In a preferred embodiment, the mixture according to one aspect of the present disclosure may comprise an organic compound according to one aspect of the disclosure and a triplet emitter.

In another preferred embodiment, the mixture according to one aspect of the present disclosure may comprise an organic compound according to one aspect of the disclosure, a triplet emitter and another triplet host material.

In a preferred embodiment, the mixture according to one aspect of the present disclosure may comprise an organic compound according to one aspect of the disclosure and two other triplet emitters.

The present disclosure further relates to a formulation which may comprise said organic compound or a mixture as described above, and at least one organic solvent. The present disclosure further provides a film prepared from a solution, comprising an organic compound according to one aspect of the present disclosure.

Examples of the organic solvents include, but are not limited to, methanol, ethanol, 2-methoxyethanol, dichloromethane, trichloromethane, chlorobenzene, o-dichlorobenzene, tetrahydrofuran, anisole, morpholine, toluene, o-xylene, m-xylene, p-xylene, 1,4-dioxahexane, acetone, methyl ethyl ketone, 1,2-dichloroethane, 3-phenoxytoluene, 1,1,1 - trichloroethane, 1,1,2,2-tetrachloroethane, ethyl acetate, butyl acetate, dimethylformamide, dimethylacetamide, dimethyl sulfoxide, tetrahydronaphthalene, naphthane, indene and/or their mixtures.

In a preferred embodiment, the formulation according to one aspect of the disclosure is a solution.

In another preferred embodiment, the formulation according to one aspect of the disclosure is a suspension.

The formulation in the examples of the present disclosure may comprise from 0.01 to 20 wt% of an organic compound according to an aspect of the present disclosure or the mixture thereof, more preferably from 0.1 to 15 wt%, more preferably from 0.2 to 10 wt%, and most preferably from 0.25 to 5 wt%.

The present disclosure also provides the use of said formulation as a coating or printing ink in the preparation of organic electronic devices, and particularly preferaby by means of printing or coating in a preparation process.

Among them, suitable printing or coating techniques may include, but are not limited to, ink-jet printing, nozzle printing, typography, screen printing, dip coating, spin coating, blade coating, roll printing, torsion printing, lithography, flexography, rotary printing, brush coating or pad printing, slit type extrusion coating, and so on. Preferred are inkjet printing, screen printing and slit type extrusion coating. The solution or suspension may additionally comprise one or more components such as surface active compounds, lubricants, wetting agents, dispersing agents, hydrophobic agents, binders, etc., for adjusting viscosity, film forming properties, improving adhesion, and the like. For more information about printing techniques and their requirements for solutions, such as solvent, concentration, viscosity, etc., see Handbook of Print Media: Technologies and Production Methods, edited by Helmut Kipphan, ISBN 3-540-67326-1.

Based on the above polymers, the present disclosure also provides use of the organic compounds as described above in an organic electronic device, which may be selected from, but not limited to, organic light emitting diodes (OLED), organic photovoltaics (OPVs), organic light emitting cells (OLEEC), organic field effect transistor (OFET), organic light emitting field effectors, organic lasers, organic spin electron devices, organic sensors, and organic plasmon emitting diodes, especially OLED.

In the embodiment of the present disclosure, the organic compounds are preferably used in a hole-transport layer, an electrion-blocking layer, a light-emitting layer, an electrion-transport layer, and a hole-transport layer, of an OLED device, wherein the light-emitting layer is particularly preferred.

The present disclosure further provides an organic electronic device which may comprise at least one polymer as described above. Typically, such an organic electronic device may comprise at least a cathode, an anode, and a functional layer between the cathode and the anode, wherein the functional layer may comprise at least one of the polymers as described above. The organic electronic devices may be selected from, but not limited to, organic light emitting diodes (OLED), organic photovoltaics (OPVs), organic light emitting cells (OLEEC), organic field effect transistor (OFET), organic light emitting field effectors, organic lasers, organic spin electron devices, organic sensors, and organic plasmon emitting diodes, especially OLED.

In a preferred embodiment, the above-described organic electronic device is an electroluminescent device, which may include a substrate, an anode, at least one light-emitting layer, and a cathode.

The substrate may be opaque or transparent. Transparent substrates may be used to make transparent light-emitting components. See, for example, Bulovic et al., Nature 1996, 380, p29, and Gu et al., Appl. Phys. Lett. 1996, 68, p2606. The substrate may be rigid or flexible. The substrate may be plastic, metal, semiconductor wafer or glass. Most preferably the substrate has a smooth surface. Substrates free of surface defects are particularly desirable. In a preferred embodiment, the substrate is flexible and may be selected from polymer films or plastic, with a glass transition temperature (Tg) of 150 °C or above, more preferably above 200°C, more preferably above 250 °C, and most preferably above 300 °C. Non-limiting examples of suitable flexible substrates are poly (ethylene terephthalate) (PET) and polyethylene glycol

(2,6-naphthalene) (PEN).

The anode may comprise a conductive metal or a metal oxide, or a conductive polymer. The anode may easily inject holes into the hole-injection layer (HIL) or the hole-transport layer (HTL) or the light-emitting layer. In one embodiment, the absolute value of the difference between the work function of the anode and the HOMO energy level or the valence band energy level of the emitter in the light-emitting layer or of the p-type semiconductor material of the HIL or HTL or the electron-blocking layer (EBL) may be smaller than 0.5 eV, more preferably smaller than 0.3 eV, and most preferably smaller than 0.2 eV. Non-limiting examples of anode materials may include, but are not limited to, Al, Cu, Au, Ag, Mg, Fe, Co, Ni, Mn, Pd, Pt, ITO, aluminum-doped zinc oxide (AZO), and the like. Other suitable anode materials are known and may be readily selected for use by one of ordinary skill in the art. The anode material may be deposited using any suitable technique, such as suitable physical vapor deposition, including RF magnetron sputtering, vacuum thermal evaporation, electron beam (e-beam), and the like. In some embodiments, the anode may be patterned. The patterned ITO conductive substrate is commercially available and may be used to fabricate the device according to the disclosure.

The cathode may comprise a conductive metal or a metal oxide. The cathode may easily inject electrons into the EIL or ETL or directly into the light-emitting layer. In one embodiment, the absolute value of the difference between the work function of the cathode and the LUMO energy level or the valence band energy level of the emitter in the light-emitting layer or of the n-type semiconductor material of the electron-injection layer (EIL) or the electron-transport layer (ETL) or the hole-blocking layer (HBL) may be smaller than 0.5 eV, more preferably smaller than 0.3 eV, and most preferably smaller than 0.2 eV In principle, all of the material that may be used as the cathode of an OLED may serve as a cathode material for the device of the present disclosure. Examples of the cathode material may include, but are not limited to, any one of Al, Au, Ag, Ca, Ba, Mg, LiF/Al, MgAg alloys, BaF2/Al, Cu, Fe, Co, Ni, Mn, Pd, Pt, ITO, or a combination thereof. The cathode material may be deposited using any suitable technique, such as suitable physical vapor deposition, including RF magnetron sputtering, vacuum thermal evaporation, electron beam (e-beam), and the like.

OLEDs may also comprise other functional layers such as hole-injection layer (HIL), hole-transport layer (HTL), electron-blocking layer (EBL), electron-injection layer (EIL), electron-transport layer (ETL), and hole-blocking layer (HBL), or a combination thereof. Materials suitable for use in these functional layers are described in detail above.

In a preferred embodiment, in the light emitting device according to one aspect of the present disclosure, the light-emitting layer thereof may comprise the organic compound according to the present disclosure.

The light emitting device according to one aspect of the present disclosure may have a light emission wavelength between 300 and 1000 nm, more preferably between 350 and 900 nm, and more preferably between 400 and 800 nm.

The present disclosure also provides the use of the organic electronic devices according to one aspect of the present disclosure in a variety of electronic devices, including, but not limited to, display devices, lighting devices, light sources, sensors, and the like.

The disclosure will now be described with reference to the preferred embodiments, but the disclosure is not to be construed as being limited to the following examples. It is to be understood that the appended claims are intended to cover the scope of the disclosure. Those skilled in the art will understand that modifications can be made to various embodiments of the disclosure with the teaching of the present disclosure, which will be covered by the spirit and scope of the claims of the disclosure.

Examples

1. Synthesis of a compound according to one aspect of the present disclosure 1) Synthesis of compounds (2-6):

①

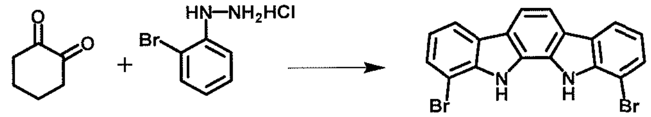

Under an argon atmosphere, 56 g (0.5 mol) of 1,2-cyclohexanedione, 224 g (1 mol) of 2-bromophenylhydrazine hydrochloride and 700 mL of ethanol were added to a 1000 mL three-necked flask. After stirring for 5min, 10 mL of concentrated sulfuric acid was slowly added, dropwise. After stirring at room temperature for 15 min, the reaction was heated to 65 ° C and reacted for 6 hours. The reaction was then stopped and the reaction solution was allowed to cool to room temperature with a large amount of solid precipitate. The reaction solution was filtered by suction, and the residue

was washed with ethanol several times. After drying, the residue was added to a 1000 mL three-necked flask, and 600 mL of acetic acid and 150 mL of trifluoroacetic acid were added. The reaction was carried out at 100 °C for 20 hours. The reaction solution was cooled to room temperature, suction filtered, and washed with acetic acid. The resulting filtrate was evaporated by rotary evaporation to give a pale yellow solid. Recrystallization was performed with a mixed solution of tetrahydrofuran/petroleum ether to gave 62 g of 1,10-dibromo-indolo [2,3a] carbazole, with a yield rate of about 30%.

②

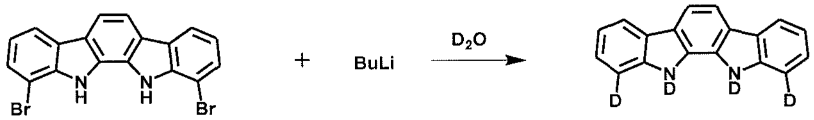


Under the argon atmosphere, 1,10-dibromo-indolo [2,3a] carbazole (31 g, 75 mmol) and 250 mL of anhydrous THF were added to a 500 mL three-necked flask, stirred to dissolve, and cooled to the temperature of -78°C with liquid nitrogen. n-butyllithium (375 mmol, 150 mL, 2.5 M n-hexane solution) was added dropwise slowly and stirred for 1.5 hours with the temperature maintained. Deuterated water ($D_2O$) (9 g, 450 mmol) was added in one portion. The reaction was allowed to warm up spontaneously and stirred at room temperature for 1 hour, and then terminated. Most of the solvent was evaporated by rotary evaporation. The reaction solution was suction filtered and the residue was recrystallized with a mixed solution of tetrahydrofuran/petroleum ether mixed solution to give 17.5 g of N, N-dideuterium -1,10- dideuterium -indolo [2,3a] carbazole in about 90% yield.

③

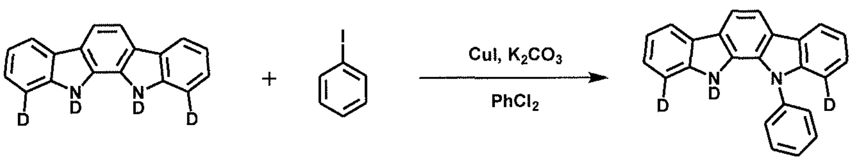


N, N- dideuterium-1,10-dideuterium-indolo [2,3a] carbazole (13 g, 50 mmol), iodobenzene (10.2 g, 50 mmol), iodide ketone (29 g,150 mmol), anhydrous potassium carbonate (21 g, 150 mmol), and 100 mL of o-dichlorobenzene were added to a 250 mL three-necked flask, and reacted at 180°C for 12 hours. The reaction solution was allowed to cool down to room temperature, function filtered, and the filtrate was distilled under reduced pressure. A pale yellow solid was produced and subjected to column chromatography with petroleum ether/dichloromethane and recrystallized in a mixed solution of tetrahydrofuran/ethanol. 13.4 g of product N-phenyl-N'-deuterium-1, 10- dideuterium-indolo [2,3a] carbazole was produced in about 80% yield.

④

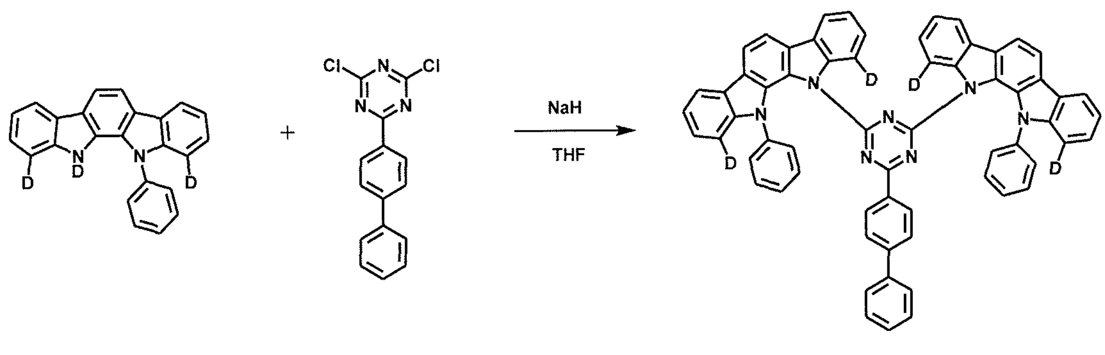


NaH solid (3.6 g, 150 mmol) was added to a 250 mL three-necked flask under argon. 150 mL of anhydrous THF was added, stirred for 10 min, N-phenyl-N'-deuterium-1, 10-dideuterium-indolo [2,3a] carbazole (10 g, 30 mmol) was slowly added and stirred at room temperature for 1 hour. 3,5-dichloro-1-biphenyl-triazine (4.5 g, 15 mmol) was

added in one portion, and continued to react for 3 days. The reaction solution was poured into ice water, stirred, and added with 300 mL of chloroform under stirring. The product was extracted. A foamy insoluble material was removed by sunction filtration. The filtrate was washed three times with water. The organic phase was dried over anhydrous magnesium sulfate, filtered and spin-dried, and recrystallized with tetrahydrofuran /ethanol to give the target compound (2-3) in a yield of 60%.

2) Synthesis of compound (3-1):

①

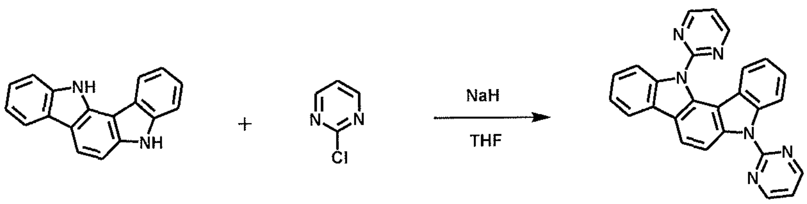


NaH solid (3.6 g, 150 mmol) was added to a 250 mL three-necked flask under argon. 150 mL of anhydrous THF was added, stirred for 10 min, indolo [3,2a] carbazole (7.7 g, 30 mmol) was slowly added and stirred at room temperature for 1 hour. 2-chloropyrimidine ( 4.5 g, 40 mmol) was added in one portion, and continued to react for 2 days. The reaction solution was poured into ice water, stirred, and added with 300 mL of chloroform under stirring. The product was extracted. A foamy insoluble material was removed by sunction filtration. The filtrate was washed three times with water. The organic phase was dried over anhydrous magnesium sulfate, filtered and spin dried, and recrystallized with tetrahydrofuran /ethanol to give N, N'-bipyrimidinylindolo [3,2a] carbazole in a yield of 80%.

②

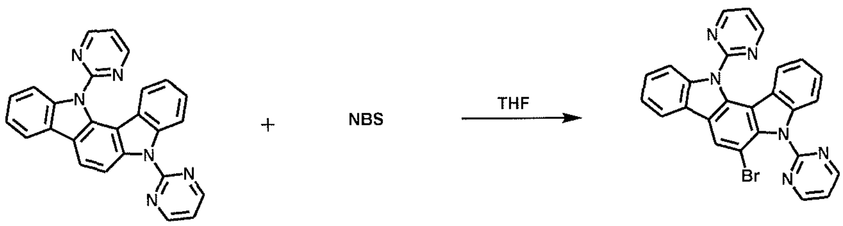


N, N'-bipyrimidinylindolo [3,2a] carbazole solid (8.2 g, 20 mmol) was added to a 150 mL three-necked flask, 50 mL of anhydrous THF was added, stirred and dissolved. N-bromosuccinimide (NBS) (3.6 g, 20 mmol) in THF (50 mL) was slowly in an ice bath. After the addition, the reaction was allowed to warm up slowly to room temperature. The reaction solution was poured into water, extracted with ethyl acetate and washed with water three times. The organic phase was dried over anhydrous magnesium sulfate, filtered and spin-dried, and recrystallized from tetrahydrofuran/ethanol to give 5-bromo-N, N'-bipyrimidinyl Benzo [3,2a] carbazole in 85% yield.

③

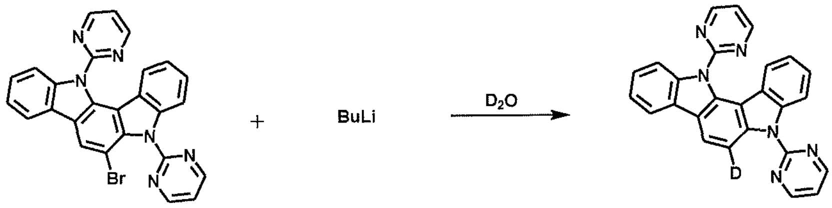

Under the argon atmosphere, 5-bromo-N, N'-bipyrimidinylindolo [3,2a] carbazole (4.9 g, 10 mmol) and 500 mL of anhydrous THF were added to a 150 mL three-necked flask and stirred to dissolve. n-butyllithium (15 mmol, 6 mL, 2.5 M n-hexane solution) was added dropwise slowly and stirred with the temperature maintaed for 1.5 hours. Deuterated water (D2O) (0.4 mL) was added dropwise at -78 °C G, 20 mmol). The reaction was allowed to warm up spontaneously and stirred at room temperature for 1 hour, and then terminated. Most of the solvent was evaporated by rotary evaporation. The reaction solution was suction filtered and the residue was recrystallized with a mixed solution of tetrahydrofuran/petroleum ether mixed solution to give 3,7 g of 5-deuterium-N, N'-bipyrimidinylindolo [3,2a] carbazole in about 90% yield.

3) As a comparison, the following is the synthesis of compound Ref1:

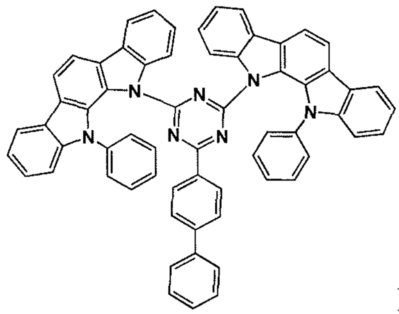

Ref1

①

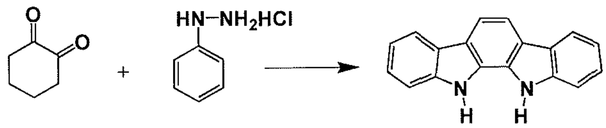

Under the argon atmosphere, 56 g (0.5 mol) of 1,2-cyclohexanedione, 144 g (1 mol) of phenylhydrazine hydrochloride, and 700 mL of ethanol were added to a 1000 mL three-necked flask and mechanically stirred for 5 min. 10 mL of concentrated sulfuric acid was slowly added, dropwise. After stirring at room temperature for 15 min, the reaction was heated to 65°C and reacted for 6 hours. The reaction was then stopped and the reaction solution was allowed to cool to room temperature with a large amount of solid precipitate. The reaction solution was filtered by suction, and the residue was washed with ethanol several times. After drying, the residue was added to a 1000 mL three-necked flask, and 600 mL of acetic acid and 150 mL of trifluoroacetic acid were added. The reaction was carried out at 100°C for 20 hours. The reaction solution was cooled to room temperature, suction filtered, and washed with acetic acid. The resulting filtrate was evaporated by rotary evaporation to give a yellow solid. Recrystallization was performed with a mixed solution of tetrahydrofuran/petroleum ether to gave 45 g of indolo [2,3a] carbazole, with a yield rate of about 35%.

②

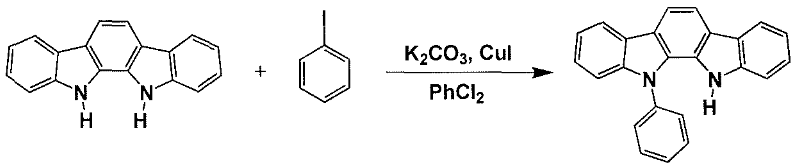


N, N- dideuterium-1,10-dideuterium-indolo [2,3a] carbazole (12.8 g, 50 mmol), iodobenzene (10.2 g, 50 mmol), iodide ketone (29 g,150 mmol), anhydrous potassium carbonate (21 g, 150 mmol), and 100 mL of o-dichlorobenzene were added to a 250 mL three-necked flask, and reacted at 180°C for 12 hours. The reaction solution was allowed to cool down to room temperature, function filtered, and the filtrate was distilled under reduced pressure. A pale yellow solid was produced and subjected to column chromatography with petroleum ether/dichloromethane and recrystallized

in a mixed solution of tetrahydrofuran/ethanol. 14.1 g of product N-phenyl-indolo [2,3a] carbazole was produced in about 85% yield.

③

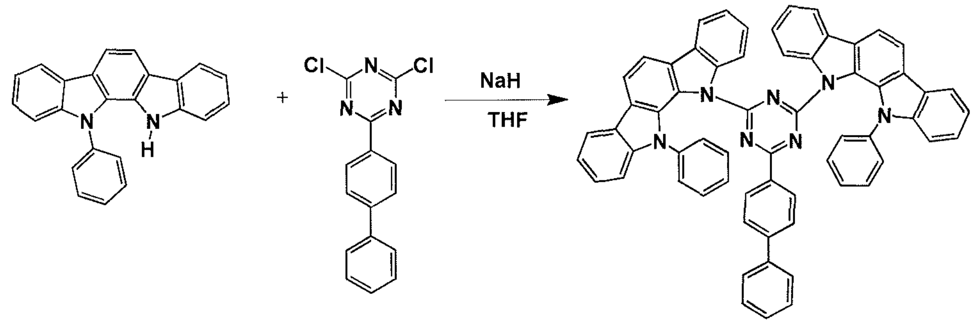


NaH solid (3.6 g, 150 mmol) was added to a 250 mL three-necked flask under argon. 150 mL of anhydrous THF was added, stirred for 10 min, N-phenyl-indolo [2,3a] carbazole (9.9 g, 30 mmol) was slowly added and stirred at room temperature for 1 hour. 3,5-dichloro-1-biphenyl-triazine (4.5 g, 15 mmol) was added in one portion, and continued to react for 3 days. The reaction solution was poured into ice water, stirred, and added with 300 mL of chloroform under stirring. The product was extracted. A foamy insoluble material was removed by sunction filtration. The filtrate was washed three times with water. The organic phase was dried over anhydrous magnesium sulfate, filtered and spin-dried, and recrystallized with tetrahydrofuran /ethanol to give the target compound in a 55% yield.

4) As a comparison, the synthesis of compound Ref2 is shown in the synthesis step in Example 2).

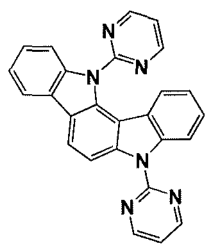

**Ref2**

2. Energy structure of organic compounds

The energy level of the organic material can be calculated by quantum computation, for example, using TD-DFT (time-dependent density functional theory) by Gaussian03W (Gaussian Inc.), the specific simulation methods of which can be found in WO2011141110. Firstly, the molecular geometry is optimized by semi-empirical method "Ground State/Semi-empirical/Default Spin/AM1" (Charge 0/Spin Singlet), and then the energy structure of organic molecules is calculated by TD-DFT (time-density functional theory) "TD-SCF/DFT/Default Spin/B3PW91" and the basis set "6-31G (d)" (Charge 0/Spin Singlet). The HOMO and LUMO levels are calculated using the following calibration formula, wherein S1 and T1 are used directly.

$$\mathrm{HOMO(eV)} = ((\mathrm{HOMO(G)} \times 27.212) - 0.9899)/1.1206$$

$$\mathrm{LUMO(eV)} = ((\mathrm{LUMO(G)} \times 27.212) - 2.0041)/1.385$$

wherein HOMO (G) and LUMO (G) are the direct calculation results of Gaussian 03W, in units of Hartree. The results are shown in Table 1:

Table 1

| Material | HOMO [eV] | LUMO [eV] | T1 [eV] | S1 [eV] |
|---|---|---|---|---|
| HATCN | -9.04 | -5.08 | 2.32 | 3.17 |
| NPB | -6.72 | -2.85 | 2.97 | 3.46 |
| TCTA | -5.34 | -2.20 | 2.73 | 3.42 |
| (2-6) | -5.95 | -2.94 | 2.73 | 3.00 |
| (3-1) | -5.92 | -2.64 | 2.80 | 3.29 |
| $Ir(ppy)_3$ | -5.30 | -2.35 | 2.70 | 2.93 |
| B3PYMPM | -5.33 | -2.20 | 2.72 | 3.28 |

Ref1 has the same energy level as (2-6) and Ref2 the same as (3-1).

3. Preparation and characterization of OLED devices

In the present example, the compounds (2-6), (3-1), Ref1 and Ref2 were used as the host materials, $Ir(ppy)_3$ as the luminescent material, HATCN as the hole-injection material, NPB and TCTA as the hole-transport Material, B3PYMPM as an electron-transport material, and the structure of the device is a electroluminescent device of ITO/HATCN/NPB/TCTA/host material: $Ir(ppy)_3$ (15%)/B3PYMPM/LiF/A1.

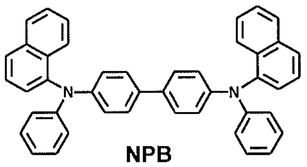

NPB

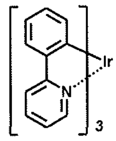

$Ir(ppy)_3$

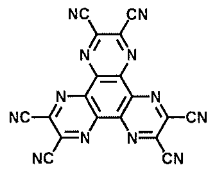

HATCN

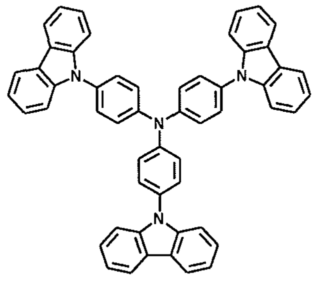

TCTA

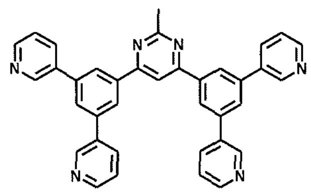

B3PYMPM

The processes for synthesis of the materials HATCN, NPB, TCTA, B3PYMPM, Ir $(ppy)_3$ are all known, which can be found in the references of the art and will not be described here.

The preparation process of the OLED device above will be described in detail with reference to specific examples below.The structure of the OLED device (as shown in Table 2) is as follows: ITO/HATCN/NPB/TCTA/host material: $Ir(ppy)_3$/B3PYMPM/LiF/A1. The preparation steps are as follows:

a, cleaning of ITO (indium tin oxide) conductive glass substrate: washing with the use of various solvents (such as one or more of chloroform, acetone or isopropyl alcohol) and then treating with UV and ozone;

b. thermal deposition in high vacuum ($1\times10^{-6}$ mbar)with HATCN (5nm), NPB (40 nm), TCTA (10nm), host material: 15% $Ir(ppy)_3$ (15 nm), B3PYMPM (40 nm), LiF (1 nm), Al (100 nm);

c, packaging: packaging the device in a nitrogen glove box with UV hardened resin.

Table 2

| OLED device | Host material |
|---|---|
| OLED1 | (2-6) |
| OLED2 | (3-1) |
| RESOLED1 | Ref1 |
| RefOLED2 | Ref2 |

The current-voltage (J-V) characteristics of each OLED device are characterized by characterization equipment, while important parameters such as efficiency, lifetime and external quantum efficiency were recorded. It was determined that the luminous efficiency of OLED1 was similar with RefOELD1 but the life of OLED1 was 2 times or higher of RefOELD1. OLED2 had a similar luminous efficiency with RefOLED2 but a life 3 times of RefOLED2. It can be seen that the life of the OLED device prepared by using the organic compound of the present disclosure is greatly improved.

## Claims

1. A compound **characterized by** having the general formula (1),

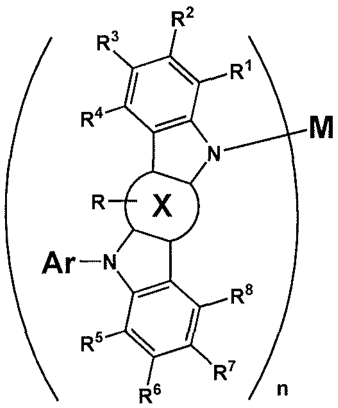

(1)

wherein,

X is an aromatic ring or a heteroaromatic ring,
R, $R^1$-$R^8$ in each occurence are the same or different, and independently selected from -H, -F, -Cl, Br, I, -D, -CN, $-NO_2$, $-CF_3$, $B(OR^0)_2$, $Si(R^0)_3$, straight chain alkane, alkane ether, alkane sulfide having 1 to 10 carbon atoms, branched alkane, cycloalkane, and alkane ether having 3 to 10 carbon atoms; R, $R^1$-$R^8$ are optionally substituted with one or more active groups $R^0$, and wherein one or more non-adjacent methylene groups may be optionally substituted with $R^0C=CR^0$, C=C, $Si(R^0)_2$, $Ge(R^0)_2$, $Sn(R^0)_2$, C=O, C=S, C=Se, $C=N(R^0)$, O, S, -COO-, or $CONR^2$; one or more H atoms of R, $R^1$-$R^8$ are optionally substituted by D, F, Cl, Br, I, CN, $NO_2$, one or more active groups $R^0$, aryl, or heteroaromatic ring;
$R^0$ in each occurrence is the same or different, and independently selected from H, D, aliphatic alkanes having 1 to 10 carbon atoms, aromatic hydrocarbon groups, and aromatic ring or heteroaromatic ring containing 5 to 10 carbon atoms;
Ar is selected from alkyl having 1 to 17 carbon atoms, cycloalkyl having 3 to 18 carbon atoms, aromatic hydrocarbon group having 6 to 60 carbon atoms, or heterocyclic aryl having 3 to 60 carbon atoms;
M is selected from aromatic hydrocarbon group having 6 to 60 carbon atoms or aromatic heterocyclic group having 3 to 60 carbon atoms; and
n is an integer of 1 to 10;
wherein at least one of R, $R^1$-$R^8$ is a D atom.

2. The compound according to claim 1, **characterized in that**, X, in multiple occurrences, is the same or different group containing a structure selected from the following:

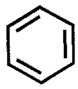
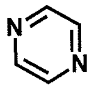
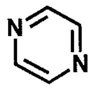
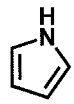
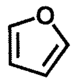
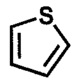

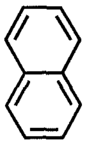
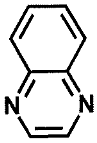
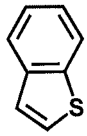
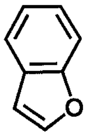
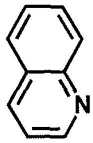

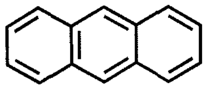
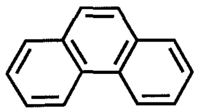
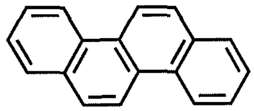
.

3. The compound according to claim 1, **characterized in that** the compound has a structure represented as any one of the following chemical formulas (2) to (7):

| | | | |
|---|---|---|---|
| 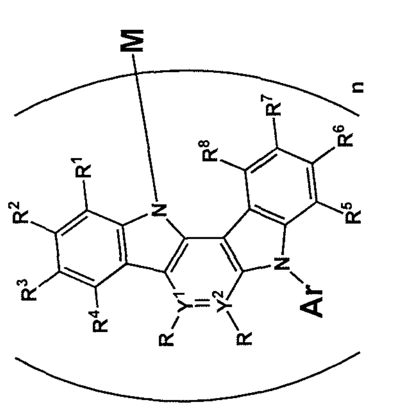 | (4) | 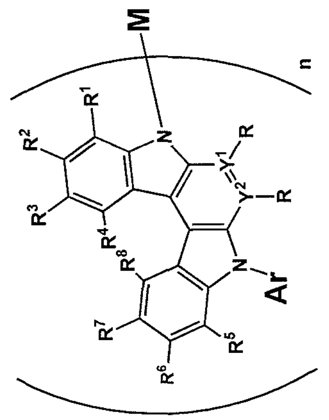 | (7) |
| 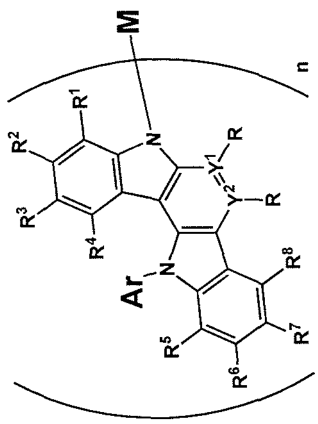 | (3) | 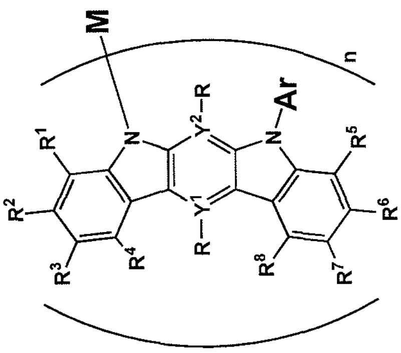 | (6) |
| 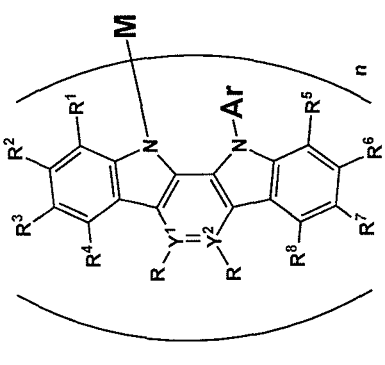 | (2) | 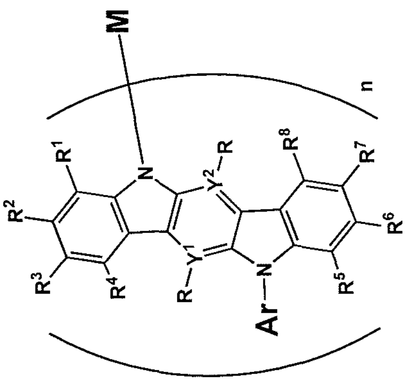 | (5) |

wherein:

$Y^1$ and $Y^2$ are selected from C or N, respectively, and when $Y^1$ and $Y^2$ are N, R is none; and
at least one of R, $R^1$-$R^8$ is a D atom, more preferably, at least two of R, $R^1$-$R^8$ are D atoms, even more preferably, at least three of R, $R^1$-$R^8$ are D atoms, and most preferably, at least four of R, $R^1$-$R^8$ are D atoms.

4. The compound according to claim 1, **characterized in that** the compound has a structure of the following chemical formula:

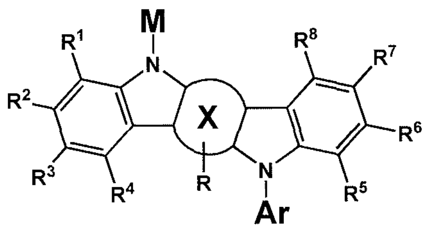

（1a）.

5. The compound according to claim 1, **characterized in that** M or Ar comprises a group with hole-transport property.

6. The compound according to claim 1, **characterized in that** M or Ar comprises a group with electron-transport property.

7. The compound according to claim 1, **characterized in that** one of M and Ar comprises a group with electron-transport property and the other comprises a group with hole-transport property.

8. A mixture **characterized by** comprising a compound according to any one of claims 1 to 7, and at least one organic functional material selected from the group consisting of: a hole-injection material, a hole-transport material, an electron-transport material, an electron-injection material, an electron-blocking material, a hole-blocking material, a light-emitting material, and a host material, or a combination thereof.

9. A formulation **characterized by** comprising a compound according to any one of claims 1 to 7, and at least one organic solvent.

10. An organic electronic device **characterized by** comprising a compound according to any one of claims 1 to 7 or a combination thereof.

11. The organic electronic device according to claim 10, **characterized in that** the organic electronic device is selected from the group consisting of: organic light emitting diode, organic photovoltaic cell, organic light emitting cell, organic field effect transistor, organic light emitting field effect transistor, organic laser, organic spin-electron device, organic sensor, and organic plasmonic emitter diode.

# INTERNATIONAL SEARCH REPORT

International application No. **PCT/CN2015/097193**

**A. CLASSIFICATION OF SUBJECT MATTER**

C07D 487/04 (2006.01) i; C07D 495/14 (2006.01) i; C07D 487/14 (2006.01) i; C07D 519/00 (2006.01) i; C07F 7/10 (2006.01) i; C07F 9/6561 (2006.01) i; C09K 11/06 (2006.01) i; H01L 51/54 (2006.01) i; H01L 51/46 (2006.01) i

According to International Patent Classification (IPC) or to both national classification and IPC

**B. FIELDS SEARCHED**

Minimum documentation searched (classification system followed by classification symbols)

C07D; C07F; C09K; H01L

Documentation searched other than minimum documentation to the extent that such documents are included in the fields searched

Electronic data base consulted during the international search (name of data base and, where practicable, search terms used)

VEN, CNABS, CPRSABS, CNKI, CA: electronic device, indole, carbazole, electrol+

**C. DOCUMENTS CONSIDERED TO BE RELEVANT**

| Category* | Citation of document, with indication, where appropriate, of the relevant passages | Relevant to claim No. |
|---|---|---|
| A | US 2014155600 A1 (SAMSUNG DISPLAY CO., LTD.), 05 June 2014 (05.06.2014), claims 1-6, description, page 5, and compound 29 | 1-11 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 01 March 2016 (01.03.2016) | **11 March 2016 (11.03.2016)** |
| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br>**HAN, Yating**<br>Telephone No.: (86-10) **62086315** |

Form PCT/ISA/210 (second sheet) (July 2009)

**INTERNATIONAL SEARCH REPORT**
Information on patent family members

International application No.
**PCT/CN2015/097193**

| Patent Documents referred in the Report | Publication Date | Patent Family | Publication Date |
|---|---|---|---|
| US 2014155600 A1 | 05 June 2014 | KR 20140070360 A | 10 June 2014 |
| | | JP 2014110315 A | 12 June 2014 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010135519 A1
- US 20090134784 A1
- WO 2011110277 A1
- US 3567450 A
- US 4720432 A
- US 5061569 A
- US 3615404 A
- US 7767317 B2
- EP 1941562 B1
- EP 1144543 B1
- WO 2006000388 A
- WO 2006058737 A
- WO 2006000389 A
- WO 2007065549 A
- WO 2007115610 A
- US 7250532 B2
- DE 102005058557 A1
- CN 1583691 A
- JP 08053397 A
- US 6251531 B1
- US 2006210830 A
- EP 1957606 A1
- US 20080113101 A1
- US 5121029 A
- WO 2006122630 A
- WO 2008006449 A
- WO 2007140847 A
- US 20060222886 A
- US 5130603 A
- US 20070092753 A1
- US 20070252517 A1
- US 4769292 A
- US 6020078 A
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- US 20070087219 A1
- US 20090061681 A1
- US 20010053462 A1
- US 6824895 B
- US 7029766 B
- US 6835469 B
- US 6830828 B
- WO 2007095118 A1
- US 2012004407 A1
- WO 2012007088 A1
- WO 2012007087 A1
- WO 2012007086 A1
- US 2008027220 A1
- WO 2011157339 A1
- CN 102282150 A
- WO 2009118087 A1
- US 7250226 B2
- JP 2007059939 A
- JP 2007211243 A
- JP 2007197574 A
- JP 2005108556 B
- JP 2005285661 B
- JP 2003338375 B
- DE 102009023154
- DE 102009023156
- WO 2011141110 A

### Non-patent literature cited in the description

- *Adv. Funct. Mater.,* 2014, vol. 24, 3551-3561
- *Chem. Rev.,* 1955, vol. 55, 713-743
- Dendrimers and Dendrons. Wiley-VCH Verlag GmbH & Co, 2002
- **BALDO ; THOMPSON et al.** *Nature,* 2000, vol. 403, 750-753
- **ADACHI et al.** *Appl. Phys. Lett.,* 2001, vol. 78, 1622-1624
- **J. KIDO et al.** *Appl. Phys. Lett.,* 1994, vol. 65, 2124
- **KIDO et al.** *Chem. Lett.,* 1990, vol. 657
- **JOHNSON et al.** *JACS,* 1983, vol. 105, 1795

- **WRIGHTON.** *JACS,* 1974, vol. 96, 998
- **MA et al.** *Synth. Metals,* 1998, vol. 94, 245
- Handbook of Print Media: Technologies and Production Methods. Kipphan
- **BULOVIC et al.** *Nature,* 1996, vol. 380, 29
- **GU et al.** *Appl. Phys. Lett.,* 1996, vol. 68, 2606